(19) Europäisches Patentamt
European Patent Office
Office européen des brevets

(11) EP 4 670 704 A2

(12) EUROPEAN PATENT APPLICATION

(43) Date of publication:
**31.12.2025 Bulletin 2026/01**

(21) Application number: **25183608.6**

(22) Date of filing: **18.06.2025**

(51) International Patent Classification (IPC):
***A61K 6/836*** (2020.01) ***A61K 6/887*** (2020.01)
***A61K 6/76*** (2020.01) ***A61K 6/77*** (2020.01)
***A61K 6/16*** (2020.01) ***A61K 6/62*** (2020.01)

(52) Cooperative Patent Classification (CPC):
(C-Sets available)
**A61K 6/887; A61K 6/16; A61K 6/62; A61K 6/76; A61K 6/77** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **25.06.2024 JP 2024102444**

(71) Applicant: **Shofu Inc.**
**Kyoto-shi, Kyoto 605-0983 (JP)**

(72) Inventors:
- **KOBAYASHI, Hiroyuki**
  **Kyoto, 605-0983 (JP)**
- **MIYATA, Shunsuke**
  **Kyoto, 605-0983 (JP)**

(74) Representative: **Eisenführ Speiser**
**Patentanwälte Rechtsanwälte PartGmbB**
**Gollierstraße 4**
**80339 München (DE)**

# (54) DENTAL COMPOSITE RESIN KIT

(57) To provide a dental composite resin kit which can reproduce a wide range of shades with a relatively small number of dental composite resins used in combination when performing aesthetic filling and restoration on teeth of various shades.

To provide a dental composite resin kit including at least a first dental composite resin and a second dental composite resin, wherein
a contrast ratio of a cured body measured at a thickness of 1 mm of the first dental composite resin is 0.3 to 0.75,
a contrast ratio of a cured body measured at a thickness of 1 mm of the second dental composite resin is 0.3 to 0.75,
a difference in contrast ratio of a cured body between the first dental composite resin and the second dental composite resin is 0.3 or less, and
when it is defined that ($L_1^*$, $a_1^*$, $b_1^*$) is a value of a color tone in a cured body having a thickness of 1 mm of the first dental composite resin in the L*a*b* space measured in the white background and ($L_2^*$, $a_2^*$, $b_2^*$) is a value of a color tone in a cured body having a thickness of 1 mm of the second dental composite resin in the L*a*b* space measured in the white background,
the color difference ΔE expressed by the following formula (1) is 2 or more.

$$\text{Formula (1): } \Delta E = \{(L_1^* - L_2^*)^2 + (a_1^* - a_2^*)^2 + (b_1^* - b_2^*)^2\}^{1/2}$$

(52) Cooperative Patent Classification (CPC): (Cont.)

C-Sets
**A61K 6/887, C08L 33/08;**
**A61K 6/887, C08L 33/10**

## Description

CROSS-REFERENCE TO RELATED APPLICATIONS

This application is based on and claims the benefit of priority from Japanese Patent Application Serial No. 2024-102444 (filed on June 25, 2024), the contents of which are hereby incorporated by reference in their entirety.

BACKGROUND OF THE INVENTION

Field of the Invention

The present invention relates to a dental composite resin.

Description of the Related Art

In the dental field, dental composite resins have been used.
In International Publication No. 2014/148293, a dental filling and restorative material kit that includes a combination of an enamel restorative material and a dentin restorative material is proposed.
In Japanese Unexamined Patent Application Publication No. 2016-175851, a dental filling and restorative material kit that includes a combination of an external filling material and a dental polymerizable composition.

SUMMARY OF THE INVENTION

Technical Problem

In these conventional kits, in order to perform aesthetic filling and restoration with respect to teeth of various shades, it has been required to prepare stocks of many composite resins having different shades. Furthermore, because the kit is designed to using a combination many composite resins and materials, there has been room for reducing the burden on operator in dental clinical practice. For example, commercially available kits offer a lineup of many shades, such as enamel shades (for upper layer filling) and body shades (for lower layer filling), and these are designed to achieve a shade similar to that of a natural tooth by filling these in the sites corresponding to the dentin and enamel in the cavity respectively.
The present invention provides a dental composite resin kit in which because the sites to which two dental composite resin to be stacked correspond is not limited to the lower or upper layer in advance, it is possible to perform aesthetic filling and restoration to teeth even in the case of switching the corresponding sites from the upper layer to the lower layer and/or from the lower layer to the upper layer depending on the shade of the tooth to be filled, and therefore it is possible to reproduce more shades than conventional kits with the same number of composite resins as conventional kits by switching the corresponding sites from the upper layer to the lower layer and/or from the lower layer to the upper layer, and to perform aesthetic filling and restoration of teeth of various shades.

Solution to Problem

In order to solve the above problems, the present inventors made an intensive study for the case of stacking two dental composite resins having different shades. As a result, the present orss have found that, by using two dental composite resins satisfying a predetermined relationship in terms of shade, it is possible to perform aesthetic filling and restoration to teeth that have different shades both before and after switching the lower and upper layers of the two dental composite resins, and therefore, by including the two dental composite resins that satisfy this predetermined relationship in a kit, it is possible to reproduce a wide range of shades and to perform aesthetic filling and restoration on teeth of various shades, even if the number of dental composite resins used together is reduced. The present invention is based on the above findings.
The present invention provides a dental composite resin kit including at least a first dental composite resin and a second dental composite resin, wherein

a contrast ratio of a cured body measured at a thickness of 1 mm of the first dental composite resin is 0.3 to 0.75, a contrast ratio of a cured body measured at a thickness of 1 mm of the second dental composite resin is 0.3 to 0.75, a difference in contrast ratio of a cured body between the first dental composite resin and the second dental composite resin is 0.3 or less, and
when it is defined that ($L_1$*, $a_1$*, $b_1$*) is a value of a color tone in a cured body having a thickness of 1 mm of the first

dental composite resin in the L*a*b* space measured in the white background and ($L_2^*$, $a_2^*$, $b_2^*$) is a value of a color tone in a cured body having a thickness of 1 mm of the second dental composite resin in the L*a*b* space measured in the white background,
the color difference ΔE expressed by the following formula (1) is 2 or more.

$$\text{Formula (1): } \Delta E = \{(L_1^* - L_2^*)^2 + (a_1^* - a_2^*)^2 + (b_1^* - b_2^*)^2\}^{1/2}$$

In the present invention, in a layered body in which a cured body having a thickness of 0.3 mm of one of the first dental composite resin and the second dental composite resin and a cured body having a thickness of 0.7 mm of the other of the first dental composite resin and the second dental composite resin are stacked,

when it is defined that ($L_{S1}^*$, $a_{S1}^*$, $b_{S1}^*$) is a value of a color tone of the layered body in the L*a*b* space measured in the white background in a state that the cured body having a thickness of 0.3 mm is positioned at front side and ($L_{S2}^*$, $a_{S2}^*$, $b_{S2}^*$) is a value of a color tone of the layered body in the L*a*b* space measured in the white background in a state that the cured body having a thickness of 0.7 mm is positioned at front side,
the lightness difference ΔL*1 expressed by the following formula (2) and the chroma difference ΔC*1 expressed by the following formula (3) may satisfy the relationship of the following formula (4) in the the first dental composite resin and the second dental composite resin.

$$\text{Formula (2): } \Delta L^*1 = |L_{S1}^* - L_{S2}^*|$$

$$\text{Formula (3): } \Delta C^*1 = |\{(a_{S1}^*)^2 + (b_{S1}^*)^2\}^{1/2} - \{(a_{S2}^*)^2 + (b_{S2}^*)^2\}^{1/2}|$$

$$\text{Formula (4): } \Delta L^*1 < \Delta C^*1$$

In the present invention, at least one of the first dental composite resin and the second dental composite resin may have a light diffusivity of 1 or more in the cured body having a thickness of 1 mm.

In the present invention, in a layered body in which a cured body having a thickness of 0.3 mm of the other of the first dental composite resin and the second dental composite resin and a cured body having a thickness of 0.7 mm of the one of the first dental composite resin and the second dental composite are stacked,

when it is defined that ($L_{S3}^*$, $a_{S3}^*$, $b_{S3}^*$) is a value of a color tone of the layered body in the L*a*b* space measured in the white background in a state that the cured body having a thickness of 0.3 mm is positioned at front side and ($L_{S4}^*$, $a_{S4}^*$, $b_{S4}^*$) is a value of a color tone of the layered body in the L*a*b* space measured in the white background in a state that the cured body having a thickness of 0.7 mm is positioned at front side,
the lightness difference ΔL*2 expressed by the following formula (5) and the chroma difference ΔC*2 expressed by the following formula (6) may satisfy the relationship of the following formula (7) in the the first dental composite resin and the second dental composite resin.

$$\text{Formula (5): } \Delta L^*2 = |L_{S3}^* - L_{S4}^*|$$

$$\text{Formula (6): } \Delta C^*2 = |\{(a_{S3}^*)^2 + (b_{S3}^*)^2\}^{1/2} - \{(a_{S4}^*)^2 + (b_{S4}^*)^2\}^{1/2}|$$

$$\text{Formula (7): } \Delta L^*2 < \Delta C^*2$$

Advantageous Effects of Invention

The dental composite resin kit of the present invention can reproduce a wide range of shades and perform aesthetic filling and restoration on teeth of various shades, even if the number of dental composite resins used together is reduced, thereby reducing the burden on the operator.

DETAILED DESCRIPTION OF THE PREFERRED EMBODIMENTS

The present invention also provides a dental composite resin kit that allows to arbitrarily select/switch the shades of the dental composite resins to be filled on the front side (labial or occlusal side) and cavity floor side (lingual or pulp side) of the restoration area in multi-layer filling of the dental composite resin.

The present invention also provides a dental composite resin kit that includes dental composite resins that have multiple shades and are tinted to a translucent to slightly opaque color similar to a natural tooth, and that allows to arbitrarily select single-color filling and/or multi-layer filling of two-color in filling the dental composite resin into the restoration area.

In the present invention, in a layered body in which a cured body having a thickness of 0.3 mm of one of the first dental composite resin and the second dental composite resin and a cured body having a thickness of 0.7 mm of the other of the first dental composite resin and the second dental composite resin are stacked,

when it is defined that ($L_{S1}$*, $a_{S1}$*, $b_{S1}$*) is a value of a color tone of the layered body in the L*a*b* space measured in the white background in a state that the cured body having a thickness of 0.3 mm is positioned at front side and ($L_{S2}$*, $a_{S2}$*, $b_{S2}$*) is a value of a color tone of the layered body in the L*a*b* space measured in the white background in a state that the cured body having a thickness of 0.7 mm is positioned at front side,
the lightness difference ΔL*1 expressed by the following formula (2) and the chroma difference ΔC*1 expressed by the following formula (3) may satisfy the relationship of the following formula (4) in the first dental composite resin and the second dental composite resin.

By satisfying this relationship, in the case of switching the lower and upper layers of the two dental composite resins during restoration, it is possible to change the chroma while suppressing changes in lightness, therefore operator can easily predict color matching and control saturation.

$$\text{Formula (2): } \Delta L^{*}1 = |L_{S1}^{*} - L_{S2}^{*}|$$

$$\text{Formula (3): } \Delta C^{*}1 = |\{(a_{S1}^{*})^2 + (b_{S1}^{*})^2\}^{1/2} - \{(a_{S2}^{*})^2 + (b_{S2}^{*})^2\}^{1/2}|$$

$$\text{Formula (4): } \Delta L^{*}1 < \Delta C^{*}1$$

In this case, in a layered body in which a cured body having a thickness of 0.3 mm of the other of the first dental composite resin and the second dental composite resin and a cured body having a thickness of 0.7 mm of the one of the first dental composite resin and the second dental composite are stacked,

when it is defined that ($L_{S3}$*, $a_{S3}$*, $b_{S3}$*) is a value of a color tone of the layered body in the L*a*b* space measured in the white background in a state that the cured body having a thickness of 0.3 mm is positioned at front side and ($L_{S4}$*, $a_{S4}$*, $b_{S4}$*) is a value of a color tone of the layered body in the L*a*b* space measured in the white background in a state that the cured body having a thickness of 0.7 mm is positioned at front side,
the lightness difference ΔL*2 expressed by the following formula (5) and the chroma difference ΔC*2 expressed by the following formula (6) may satisfy the relationship of the following formula (7) in the first dental composite resin and the second dental composite resin.

$$\text{Formula (5): } \Delta L^{*}2 = |L_{S3}^{*} - L_{S4}^{*}|$$

$$\text{Formula (6): } \Delta C^{*}2 = |\{(a_{S3}^{*})^2 + (b_{S3}^{*})^2\}^{1/2} - \{(a_{S4}^{*})^2 + (b_{S4}^{*})^2\}^{1/2}|$$

$$\text{Formula (7): } \Delta L^{*}2 < \Delta C^{*}2$$

In the present invention, at least one of the first dental composite resin and the second dental composite resin may have a light diffusivity of 1 or more in the cured body having a thickness of 1 mm.

In the present invention, the first dental composite resin and the second dental composite resin may include (A) polymerizable monomer, (B) photosensitizer, (C) photoacid generator, (D) photopolymerization accelerator, (E) filler, and a colorant respectively.

Hereinafter, the dental composite resin of the present invention is described in detail.

The dental composite resin of the present invention is applied as a dental adhesive material, a dental core build-up material, a dental resin cement, a dental coating material, a dental sealant material, a dental manicure material, a dental splinting material, a dental hard resin, a dental CAD-CAM restoration material, a dental 3D printer material and the like.

In a dental practice, in order to restore aesthetically and functionally a lost portion of a tooth by caries, breakages and the like, a direct method which performs restoration with a dental adhesive material and composite resin and an indirect method which restores a prosthesis device consisting of ceramics or dental hard resin with a dental resin cement have been performed as treatment. In addition, a dental adhesive material for mounting a dental composite resin and various dental materials and a natural tooth, a dental splinting material for fixing a mobile tooth, a dental coating material for protecting a vital tooth after forming against a hyperesthesia, an external stimulation and secondary caries, a dental sealant material for preventing caries by filling deep fissures of a molar tooth, a dental manicure material for temporary recovering aesthetic property by masking discoloration of a tooth, and a dental core build-up material for forming an abutment tooth in the case of collapsing of a dental crown due to caries have been used. In recent years, new composite materials such as a dental CAD-CAM restoration material for preparing a prosthesis device by CAD/CAM processing and a dental 3D printer material for preparing a prosthesis device by 3D printer have been developed, and various dental materials have been used for treatment. he above-described materials are prepared into a uniform paste by mixing a resin matrix consisting of several kinds of polymerizable monomers, a filler such as an inorganic filler and an organic-inorganic composite filler, and a polymerization initiator, according to the application. As one example of some materials, a dental composite resin for filling is used by filling into a tooth in the form of uncured paste, imparting anatomical form of a natural tooth with a dental instrument such as an instrument, and curing by irradiating light with a dental light irradiator or the like. For the irradiation light from a light irradiator, a light source having a light intensity of about 100 to 2000 $mW/cm^2$ in a wavelength range of about 360 to 500 nm is generally used. On the other hand, a dental resin cement is used for adhering a prosthesis device to a tooth cavity or an abutment tooth, and is cured by light irradiation after attaching the prosthesis device to the tooth cavity or the abutment tooth.

As the photopolymerization initiator used for such dental materials, a system in which a photosensitizer and a photosensitizer are combined with an appropriate photopolymerization accelerator has been widely used. As the photosensitizer, acylphosphine oxide compounds and α-diketone compounds are known, and in particular, α-diketone compounds have an ability to initiate polymerization in the wavelength range of visible light which has little effect on the human body. Further, as a compound to be combined with a photosensitizer, a photoacid generator and a tertiary amine compound are well known.

On the other hand, in the dental composite resin, it is need to adapt to various shapes according to the complex shapes of teeth. Furthermore, matching the shade of a dental composite resin with the tooth to achieve aesthetic restoration contributes to improving patients QOL.

However, because tooth shade varies from patient to patient, and for dentists, preparing stocks of many composite resins having different shades and using a material in which shade is designed to using a combination many composite resins and materials are elements constituting burden, and there has been a room for improvement.

[(A) Polymerizable monomer]

As the (A) polymerizable monomer contained in the dental composite resin of the present invention, any polymerizable monomers can be used without limitation as long as it is known. In the polymerizable monomer or the compound having a polymerizable group described in the present invention, the polymerizable group preferably exhibits radical polymerizability, and specifically, from the viewpoint of easy radical polymerization, the polymerizable group is preferably (meth) acrylic group and/or (meth) acrylamide group. In the present specification, "(meth) acrylic" means acrylic and/or methacrylic, "(meth) acryloyl" means acryloyl and/ or methacryloyl, "(meth) acrylate" means acrylate and/or methacrylate, and, "(meth) acrylamide" means acrylamide and/or methacrylamide. A polymerizable monomer having a substituent at the α-position of an acrylic group and/or an acrylamide group can also be preferably used. Specific examples include one having one radical polymerizable group, one having two radical polymerizable groups, one having three or more radical polymerizable groups, one having an acidic group, one having an alkoxysilyl group, and one having a sulfur atom.

Specific examples of a polymerizable monomer having one radical polymerizable group and not containing acidic group include 2-hydroxyethyl (meth) acrylate, 3-hydroxypropyl (meth) acrylate, 4-hydroxybutyl (meth) acrylate, 2-hydroxypropyl (meth) acrylate, 2-hydroxybutyl (meth) acrylate, 6-hydroxyhexyl (meth) acrylate, 10-hydroxydecyl (meth) acrylate, propylene glycol mono (meth) acrylate, glycerol mono (meth) acrylate, erythritol mono (meth) acrylate, N-methylol (meth) acrylamide, N-hydroxyethyl (meth) acrylamide, N,N-(dihydroxyethyl) (meth) acrylamide, methyl (meth) acrylate, ethyl (meth) acrylate, propyl (meth) acrylate, isopropyl (meth) acrylate, butyl (meth) acrylate, isobutyl (meth) acrylate, benzyl (meth) acrylate, lauryl (meth) acrylate, 2,3-dibromopropyl (meth) acrylate, 3-(meth) acryloyloxypropyl trimethoxysilane, 11-(meth) acryloyloxyundecyl trimethoxysilane, (meth) acrylamide and the like.

Specific Examples of the polymerizable monomer having two radical polymerizable groups and not containing

acidic group include 2,2-bis ((meth) acryloyloxy phenyl) propane, 2,2-bis [4-(3-(meth) acryloyloxy)-2-hydroxy propoxyphenyl] propane (generally called "Bis-GMA"), 2,2-bis (4-(meth) acryloyloxy phenyl) propane, 2,2-bis (4-(meth) acryloyloxy polyethoxyphenyl) propane, 2,2-bis (4-(meth) acryloyloxy diethoxyphenyl) propane, 2,2-bis (4-(meth) acryloyloxy tetraethoxyphenyl) propane, 2,2-bis (4-(meth)) acryloyloxy pentaethoxyphenyl) propane, 2,2-bis (4-(meth) acryloyloxy dipropoxyphenyl) propane, 2-(4-(meth) acryloyloxy diethoxyphenyl)-2-(4-(meth) acryloyloxy diethoxyphenyl) propane, 2-(4-(meth) acryloyloxy diethoxyphenyl)-2-(4-(meth) acryloyloxy ditriethoxyphenyl) propane, 2-(4-(meth) acryloyloxy dipropoxyphenyl)-2-(4-(meth) acryloyloxy triethoxyphenyl) propane, 2,2-bis (4-(meth) acryloyloxy propoxyphenyl) propane, 2,2-bis (4-(meth) acryloyloxy isopropoxyphenyl) propane, 1,4-bis (2-(meth) acryloyloxyethyl) pyromellitate, glycerol di (meth) acrylate, 1-(acryloyloxy)-3-(methacryloyloxy)-2-propanol, ethyleneglycol di (meth) acrylate, diethyleneglycol di (meth) acrylate, triethylene glycol di (meth) acrylate, propylene glycol di (meth) acrylate, butylene glycol di (meth) acrylate, neopentyl glycol di (meth) acrylate, polyethylene glycol di (meth) acrylate, 1,3-butanediol di (meth) acrylate, 1,5-pentanediol di (meth) acrylate, 1,6-hexanediol di (meth) acrylate, 1,10-decanediol di (meth) acrylate, 1,2-bis (3-methacryloyloxy-2-hydroxypropoxy) ethane, 2,2,4-trimethyl hexamethylene bis (2-carbamoyloxy ethyl) dimethacrylate (generally called "UDMA"), 1,2-bis (3-methacryloyloxy-2-hydroxy propoxy) ethane and the like.

Specific Examples of the polymerizable monomer having three or more radical polymerizable groups and not containing acidic group include trimethylolpropane tri (meth) acrylate, trimethylolethane tri (meth) acrylate, trimethylolmethane tri (meth) acrylate, pentaerythritol tri (meth) acrylate, pentaerythritol tetra (meth) acrylate, dipentaerythritol penta (meth) acrylate, N,N-(2,2,4-trimethyl hexamethylene) bis [2- (aminocarboxy) propane-1,3-diol] tetra methacrylate, 1,7-diacryloyloxy-2,2,6,6-tetra acryloyloxymethyl-4-oxyheptane and the like.

For the polymerizable monomer having an acidic group, any polymerizable monomer can be used without any limitation as long as it has one or more polymerizable group and at least one acidic group such as a phosphoric acid group, a pyrophosphoric acid group, a thiophosphoric acid group, a phosphonic acid group, a sulfonic acid group and a carboxylic acid group and the like. It is possible to impart adhesive property with respect to a tooth substance and a prosthesis device by containing a polymerizable monomer having an acidic group.

Specific examples of a phosphoric acid group-containing polymerizable monomer include 2-(meth) acryloyloxyethyl dihydrogen phosphate, 3-(meth) acryloyloxypropyl dihydrogen phosphate, 4-(meth) acryloyloxybutyl dihydrogen phosphate, 5-(meth) acryloyloxypentyl dihydrogen phosphate, 6-(meth) acryloyloxyhexyl dihydrogen phosphate, 7-(meth) acryloyloxyheptyl dihydrogen phosphate, 8-(meth) acryloyloxyoctyl dihydrogen phosphate, 9-(meth) acryloyloxynonyl dihydrogen phosphate, 10-(meth) acryloyloxydecyl dihydrogen phosphate, 11-(meth) acryloyloxyundecyl dihydrogen phosphate, 12-(meth) acryloyloxydodecyl dihydrogen phosphate, 16-(meth) acryloyloxyhexadecyl dihydrogen phosphate, 20-(meth) acryloyloxyicosyl dihydrogen phosphate, bis [2-(meth) acryloyl oxyethyl] hydrogensphosphate, bis [4-(meth) acryloyl oxybutyl] hydrogen phosphate, bis [6-(meth) acryloyl oxyhexyl] hydrogen phosphate, bis [8-(meth) acryloyl oxyoctyl] hydrogen phosphate, bis [9-(meth) acryloyl oxynonyl] hydrogen phosphate, bis [10-(meth) acryloyl oxydecyl] hydrogen phosphate, 1,3-di (meth) acryloyl oxypropyl dihydrogenphosphate, 2-(meth) acryloyl oxyethylphenyl hydrogen phosphate, 2-(meth) acryloyloxyethyl-2-bromoethyl hydrogen phosphate and bis [2-(meth) acryloyloxy-(1-hyrdoxymethyl) ethyl] hydrogen phosphate; acyl chloride, alkali metal salt and ammonium salt thereof; and (meth) acrylamide compound in which the ester bond of these compounds is substituted with an amide bond, and the like.

Specific examples of a pyrophosphoric acid group-containing polymerizable monomer include bis [2-(meth) acryloyl oxyethyl] pyrophosphate, bis [4-(meth) acryloyl oxybutyl] pyrophosphate, bis [6-(meth) acryloyl oxyhexyl] pyrophosphate, bis [8-(meth) acryloyl oxyoctyl] pyrophosphate, bis [10-(meth) acryloyl oxydecyl] pyrophosphate; acyl chloride, alkali metal salt and ammonium salt thereof; and (meth) acrylamide compound in which the ester bond of these compounds is substituted with an amide bond, and the like.

Specific examples of a thiophosphate group-containing polymerizable monomer include 2-(meth) acryloyloxyethyl dihydrogen thiophosphate, 3-(meth) acryloyloxypropyl dihydrogen thiophosphate, 4-(meth) acryloyloxybutyl dihydrogen thiophosphate, 5-(meth) acryloyloxypentyl dihydrogen thiophosphate, 6-(meth) acryloyloxyhexyl dihydrogen thiophosphate, 7-(meth) acryloyloxyheptyl dihydrogen thiophosphate, 8-(meth) acryloyloxyoctyl dihydrogen thiophosphate, 9-(meth) acryloyloxynonyl dihydrogen thiophosphate, 10-(meth) acryloyloxydecyl dihydrogen thiophosphate, 11-(meth) acryloyloxyundecyl dihydrogen thiophosphate, 12-(meth) acryloyloxydodecyl dihydrogen thiophosphate, 16-(meth) acryloyloxyhexadecyl dihydrogen thiophosphate, 20-(meth) acryloyloxyicosyl dihydrogen thiophosphate; acyl chloride, alkali metal salt and ammonium salt thereof; and (meth) acrylamide compound in which the ester bond of these compounds is substituted with an amide bond, and the like. The polymerizable monomer having a thiophosphate group is also classified as a polymerizable monomer having a sulfur atom.

Specific examples of a phosphonic acid group-containing polymerizable monomer include 2-(meth) acryloyloxy ethylphenyl phosphonate, 5-(meth) acryloyloxy pentyl-3-phosphonopropionate, 6-(meth) acryloyloxy hexyl-3-phosphonopropionate, 10-(meth) acryloyloxy decyl-3-phosphonopropionate, 6-(meth) acryloyloxy hexyl-3-phosphonoacetate, 10-(meth) acryloyloxy decyl-3-phosphonoacetate; acyl chloride, alkali metal salt and ammonium salt thereof; and (meth) acrylamide compound in which the ester bond of these compounds is substituted with an amide bond, and the like.

Specific examples of a sulfonic acid group-containing polymerizable monomer include 2-(meth) acrylamide-2-methyl propanesulfonic acid and 2-sulfoethyl (meth) acrylate and the like.

The carboxylic acid group-containing polymerizable monomers are classified into a (meth) acrylic-based compound having one carboxyl group in the molecule and a (meth) acrylic-based compound having a plurality of carboxyl groups in the molecule. Examples of the (meth) acrylic-based compound having one carboxyl group in the molecule include (meth) acrylic acid, N-(meth) acryloyl glycine, N-(meth) acryloyl aspartic acid, O-(meth) acryloyl tyrosine, N-(meth) acryloyl tyrosine, N-(meth) acryloyl phenylalanine, N-(meth) acryloyl-p-aminobenzoic acid, N-(meth) acryloyl-o-aminobenzoic acid, p-vinylbenzoic acid, 2-(meth) acryloyloxybenzoic acid, 3-(meth) acryloyloxybenzoic acid, 4-(meth) acryloyloxybenzoic acid, N-(meth) acryloyl-5-aminosalicylic acid, N-(meth) acryloyl-4-aminosalicylic acid, 2-(meth) acryloyloxyethyl hydrogen succinate, 2-(meth) acryloyloxyethyl hydrogen phthalate, 2-(meth) acryloyloxyethyl hydrogenmalate; acyl chloride thereof; and (meth)acrylamide compound in which the ester bond of these compounds is substituted with an amide bond, and the like. Examples of the (meth) acrylic-based compound having a plurality of carboxyl groups in the molecule include 6-(meth) acryloyl oxyhexane-1,1-dicarboxylic acid, 9-(meth) acryloyl oxynonane-1,1-dicarboxylic acid, 10-(meth) acryloyl oxydecane-1,1-dicarboxylic acid, 11-(meth) acryloyloxy undecane-1,1-dicarboxylic acid, 12-(meth) acryloyl oxydodecane-1,1-dicarboxylic acid, 13-(meth) acryloyloxy tridecane-1,1-dicarboxylic acid, 4-(meth) acryloyloxyethyl trimeritate, 4-(meth) acryloyloxybutyl trimeritate, 4-(meth) acryloyloxyhexyl trimeritate, 4-(meth) acryloyloxydecyl trimeritate, 2-(meth) acryloyl oxyethyl-3'-(meth) acryloyloxy-2'-(3,4-dicarboxy benzoyloxy) propylsuccinate; acid anhydrides and acid halides thereof,; and (meth) acrylamide compound in which the ester bond of these compounds is substituted with an amide bond, and the like.

Preferable examples include 10-methacryloyloxydecyl dihydrogen phosphate and 6-methacryloxyhexyl phosphonoacetate. From the view point of imparting adhesive property, the compounding amount of the polymerizable monomer having an acidic group is preferably 1 part by mass or more, more preferably 1 part by mass or more and 30 parts by mass or less with respect to 100 parts by mass of total amount of the polymerizable monomer contained in the dental composite resin. When the compounding amount is less than 1 part by mass, there is a case that the adhesive property to the tooth substance, metal and metal oxides is not exhibited sufficiently. When the compounding amount is more than 30 parts by mass, there is a case that a decrease in storage stability occurs.

Specific examples of the polymerizable monomer having an alkoxysilyl gruop include a (meth) acrylic compound and a (meth) acrylamide compound having one alkoxysilyl group in the molecule and a (meth) acrylic compound and a (meth) acrylamide compound having a plurality of alkoxysilyl groups in the molecule. Specific examples include 2-(meth) acryloxyethyl trimethoxysilane, 3-(meth) acryloxypropyl trimethoxysilane, 3-(meth) acryloxypropyl triethoxysilane, 3-(meth) acryloxypropyl methyldimethoxysilane, 4-(meth) acryloxybutyl trimethoxysilane, 5-(meth) acryloxypentyl trimethoxysilane, 6-(meth) acryloxyhexyl trimethoxysilane, 7-(meth) acryloxyheptyl trimethoxysilane, 8-(meth) acryloxyoctyl trimethoxysilane, 9-(meth) acryloxynonyl trimethoxysilane, 10-(meth) acryloxydecyl trimethoxysilane, 11-(meth) acryloxyundecyl trimethoxysilane. Furthermore, specific examples having an urethane group or an ether group include 3,3-dimethoxy-8,37-dioxo-2,9,36-trioxa-7,38-diaza-3-silatetracontan-40-yl (meth) acrylate, 2-((3,3-dimethoxy-8-oxo-2,9,18-trioxa-7-aza-3-silanonadecane-19-oyl) amino)-2-methylpropane-1,3-diyl di (meth) acrylate, 3,3-dimethoxy-8,19-dioxo-2,9,18-trioxa-7,20-diaza-3-siladocosane-22-yl (meth) acrylate, 3,3-dimethoxy-8,22-dioxo-2,9,12,15,18,21-hexaoxa-7,23-diaza-3-silapentacosane-25-yl (meth) acrylate, 3,3-dimethoxy-8,22-dioxo-2,9,12,15,18,21,26-heptaoxa-7,23-diaza-3-silaoctacosane-28-yl (meth) acrylate, 3,3-dimethoxy-8,19-dioxo-2,9,12,15,18-pentaoxa-7,20-diaza-3-siladocosane-22-yl (meth) acrylate, 3,3-dimethoxy-8,19-dioxo-2,9,12,15,18,23-hexaoxa-7,20-diaza-3-silapentacosane-25-yl (meth) acrylate, 2-((3,3-dimethoxy-8-oxo-2,9,12,15,18-pentaoxa-7-aza-3-silanonadecane-19-oyl) amino) -2-methylpropan-1,3-diyldi (meth) acrylate, 4,4-diethoxy-17-oxo-3,16,21-trioxa-18-aza-4-silatricosane-23-yl (meth) acrylate, 4,4-diethoxy-17-oxo-3,16,21,24-tetraoxa-18-aza-4-silahexacosane-26-yl (meth) acrylate, 4,4-diethoxy-13-oxo-3,12,17-trioxa-14-aza-4-silanonadecane-19-yl (meth) acrylate, 4,4-diethoxy-17-oxo-3,16-dioxa-18-aza-4-silaicosane-20-yl (meth) acrylate and 2-methyl-2-((11-(triethoxysilyl) undecyloxy) carbonylamino) propan-1,3-diyldi (meth) acrylate.

The dental composite resin of the present invention may contain a polymerizable monomer having a sulfur atom as the (A) polymerizable monomer in order to impart adhesive property with respect to a noble metal. As the polymerizable monomer having a sulfur atom, any known compound can be used without any limitation as long as it is a polymerizable monomer having one or more sulfur atoms and a polymerizable group. Specifically, it refers to a compound having a partial structure such as -SH, -S-S-, >C=S, >C-S-C<, >P=S, or a compound prepared by tautomerism thereof. Specific examples include 10-methacryloxy decyl-6,8-dithiooctanate, 6-methacryloxy hexyl-6,8-dithiooctanate, 6-methacryloxy hexyl-2-thiouracil-5-carboxylate, 2-(11-methacryloxy undecylthio)-5-mercapto-1,3,4-thiadiazole, 10-(meth) acryloxy decyl dihydrogenthiophosphate.

An oligomer or a prepolymer having at least one polymerizable group in its molecule may be used other than such a polymerizable monomer, without any limitation. There is no problem even if a substituent such as a fluoro group is contained in the same molecule. The polymerizable monomers described above can be used not only singly but also in combinations of a plurality thereof.

The dental composite resin of the present invention may contain a silane coupling agent as the (A) polymerizable

monomer in order to impart adhesive property with respect to glass ceramics. Any known silane coupling agent can be used without any limitation, but 3-methacryloxypropyl trimethoxysilane, 8-methacryloxyoctyl trimethoxysilane, and 11-methacryloxyundecyl trimethoxysilane and the like are preferable. From the viewpoint of imparting adhesive property, the compounding amount is, with respect to 100 parts by mass of the total amount of the polymerizable monomer contained in the composition, preferably 1 part by mass or more, more preferably 5 parts by mass or more and less than 20 parts by mass. Since the purpose of the silane coupling agent as a polymerizable monomer is to impart adhesive property with respect to glass ceramics or a resin material containing a filler consisting of glass ceramics, the silane coupling agent is compounded separately from the surface treatment agent of the filler.

The dental composite resin of the present invention may contain a polymerizable monomer having a sulfur atom as the (A) polymerizable monomer in order to impart adhesive property with respect to a noble metal. From the view point of imparting adhesive property, the compounding amount of the polymerizable monomer having a sulfur atom is, with respect to 100 parts by mass of the total amount of the polymerizable monomer contained in the composite resin, 0.01 parts by mass or more, preferably 0.1 parts by mass or more and less than 20 parts by mass.

<Photopolymerization initiator>

The dental composite resin of the present invention includes, as a photopolymerization initiator, (B) photosensitizer and (D) photopolymerization accelerator, and may include (C) photoacid generator. These are not particularly limited, and any known compounds commonly used may be used without any limitation.

[(B) Photosensitizer]

Specific examples of the (B) photosensitizer which can be used in the present invention include α-diketones such as benzil, camphorquinone, camphorquinone carboxylic acid, camphorquinone sulfonic acid, α-naphthyl, acetonaphthene, p,p'-dimethoxybenzyl, p,p'-dichlorobenzylacetyl, pentanedion, 1,2-phenanthrenequinone, 1,4-phenanthrenequinone, 3,4-phenanthrenequinone, 9,10-phenanthrenequinone and naphthoquinone; benzoin alkyl ethers such as benzoin, benzoin methyl ether and benzoin ethyl ether; thioxanthones such as thioxanthone, 2-chlorothioxanthone, 2-methylthioxanthone, 2-isopropylthioxanthone, 2-methoxythioxanthone, 2-hydroxythioxanthone, 2,4-diethylthioxanthone and 2,4-diisopropylthioxanthone; benzophenones such as benzophenone, p-chlorobenzophenone and p-methoxybenzophenone; acylphosphine oxides such as bis (2,6-dimethoxy benzoyl) phenylphosphine oxide, bis (2,6-dimethoxy benzoyl) (2,4,4-trimethyl pentyl) phosphine oxide, bis (2,6-dimethoxy benzoyl)-n-butylphosphine oxide, bis (2,6-dimethoxy benzoyl)-(2-methylprop-1-yl) phosphine oxide, bis (2,6-dimethoxy benzoyl)-(1-methylprop-1-yl) phosphine oxide, bis (2,6-dimethoxy benzoyl)-t-butyl phosphine oxide, bis (2,6-dimethoxy benzoyl) cyclohexyl phosphine oxide, bis (2,6-dimethoxy benzoyl) octyl phosphine oxide, bis (2-methoxy benzoyl) (2-methylprop-1-yl) phosphine oxide, bis (2-methoxy benzoyl) (1-methylprop-1-yl) phosphine oxide, bis (2,6-diethoxy benzoyl) (2-methylprop-1-yl) phosphine oxide, bis (2,6-diethoxy benzoyl) (1-methylprop-1-yl) phosphine oxide, bis (2,6-dibutoxy benzoyl) (2-methylprop-1-yl) phosphine oxide, bis (2,4-dimethoxy benzoyl) (2-methylprop-1-yl) phosphine oxide, bis (2,4,6-trimethyl benzoyl) phenyl phosphine oxide, 2,4,6-trimethyl benzoyl diphenyl phosphine oxide, bis (2,4,6-trimethyl benzoyl) (2,4-dipentoxy phenyl) phosphine oxide, bis (2,6-dimethoxy benzoyl) benzyl phosphine oxide, bis (2,6-dimethoxy benzoyl)-2-phenylpropyl phosphine oxide, bis (2,6-dimethoxy benzoyl)-2-phenylethyl phosphine oxide, bis (2,6-dimethoxy benzoyl) benzyl phosphine oxide, bis (2,6-dimethoxy benzoyl)-2-phenylpropyl phosphine oxide, bis (2,6-dimethoxy benzoyl)-2-phenylethyl phosphine oxide, 2,6-dimethoxy benzoyl benzyl butyl phosphine oxide, 2,6-dimethoxy benzoyl benzyl octyl phosphine oxide, bis (2,4,6-trimethyl benzoyl) isobutyl phosphine oxide and 2,6-dimethoxy benzoyl-2,4,6-trimethyl benzoyl-n-butyl phosphine oxide; acylgermanium compounds such as bisbenzoyl diethylgermanium, bisbenzoyl dimethylgermanium, bisbenzoyl dibutylgermanium, bis (4-methoxybenzoyl) dimethylgermanium and bis (4-methoxybenzoyl) diethylgermanium; α-aminoacetophenones such as 2-benzyl-dimethylamino-1-(4-morpholinophenyl)-butanone-1, and 2-benzyl-diethylamino-1-(4-morpholinophenyl)-propanone-1; ketals such as benzyl dimethyl ketal, benzyl diethyl ketal and benzyl (2-methoxyethyl ketal); and titanocenes such as bis (cyclopentadienyl)-bis [2,6-difluoro-3-(1-pyrrolyl) phenyl]-titanium, bis (cyclopentadienyl)-bis (pentanefluorophenyl)-titanium and bis (cyclopentadienyl)-bis (2,3,5,6-tetrafluoro-4-disiloxyphenyl)-titanium.

The photosensitizer (B) may be appropriately selected according to the wavelength, the intensity and the irradiation time of light used for polymerization, and the type and the compounding amount of other components to be combined. In addition, the photosensitizer may be used not only singly but also in combinations of two or more. Among them, α-diketone compounds having a maximum absorption wavelength in the visible light region are preferably used, and camphorquinone compounds such as camphorquinone, camphorquinone carboxylic acid and camphorquinone sulfonic acid are more preferable. Camphorquinone is particularly preferred because it is easily available.

Usually, the compounding amount of the (B) photosensitizer with respect to 100 parts by mass of the total amount of the (A) polymerizable monomer contained in the dental composite resin is preferably 0.02 to 1 parts by mass, more preferably 0.05 to 0.5 parts by mass. When the compounding amount of the (B) photosensitizer is less than 0.02 parts by

mass, there is a case that the polymerization activity with respect to the irradiation light is poor and the curing becomes insufficient. When the compounding amount is more than 1 parts by mass, there is a case that although sufficient curability is exhibited, the sensitivity to light is low, and yellowness is increased.

The dental composite resin of the present invention may contain only the (B-1) α-diketone compounds as the (B) photosensitizer.

[(C) Photoacid generator]

As the (C) photoacid generator can be used in the dental composite resin of the present invention, known compounds can be used without limitation. Specific examples include triazine compounds, iodonium salt compounds, sulfonium salt compounds, and sulfonic acid ester compounds. Among these, triazine compounds and iodonium salt-based compounds are preferable because of having high polymerizability in the case of using in combination with a sensitizer. Iodonium salt-based compounds are more preferable. Iodonium-based salt compounds are susceptible to sensitization by photosensitizers that have absorption in the visible light region.

Specific examples of the triazine compound include 2,4,6-tris (trichloro methyl)-s-triazine, 2,4,6-tris (tribromo methyl)-s-triazine, 2-methyl-4,6-bis (trichloro methyl)-s-triazine, 2-methyl-4,6-bis (tribromo methyl)-s-triazine, 2-phenyl-4,6-bis (trichloro methyl)-s-triazine, 2-(p-methoxy phenyl)-4,6-bis (trichloro methyl)-s-triazine, 2-(p-methyl thiophenyl)-4,6-bis (trichloro methyl)-s-triazine, 2-(p-chloro phenyl)-4,6-bis (trichloro methyl)-s-triazine, 2-(2,4-dichloro phenyl)-4,6-bis (trichloro methyl)-s-triazine, 2-(p-bromo phenyl)-4,6-bis (trichloro methyl)-s-triazine, 2-(p-tolyl)-4,6-bis (trichloro methyl)-s-triazine, 2-n-propyl-4,6-bis (trichloro methyl)-s-triazine, 2-(α,α,β-trichloro ethyl)-4,6-bis (trichloro methyl)-s-triazine, 2-styryl-4,6-bis (trichloro methyl)-s-triazine, 2-[2-(p-methoxy phenyl) ethenyl]-4,6-bis (trichloro methyl)-s-triazine, 2-[2-(o-methoxy phenyl) ethenyl]-4,6-bis (trichloro methyl)-s-triazine, 2-[2-(p-butoxy phenyl) ethenyl]-4,6-bis (trichloro methyl)-s-triazine, 2-[2-(3,4-dimethoxy phenyl) ethenyl]-4,6-bis (trichloro methyl)-s-triazine, 2-[2-(3,4,5-trimethoxy phenyl) ethenyl]-4,6-bis (trichloro methyl)-s-triazine, 2-(1-naphthyl)-4,6-bis (trichloro methyl)-s-triazine, 2-(4-biphenylyl)-4,6-bis (trichloro methyl)-s-triazine, 2-[2-{N,N-bis(2-hydroxy ethyl) amino} ethoxy]-4,6-bis (trichloro methyl)-s-triazine, 2-[2-{N-hydroxy ethyl-N-ethylamino} ethoxy]-4,6-bis (trichloro methyl)-s-triazine, 2-[2-{N-hydroxy ethyl-N-methylamino} ethoxy]-4,6-bis (trichloro methyl)-s-triazine, 2-[2-{N,N-diallyl amino} ethoxy]-4,6-bis (trichloro methyl)-s-triazine and the like. Among them, 2,4,6-tris (trichloro methyl)-s-triazine is preferable.

Any iodonium salt-based compound can be used as long as it is known. For the specific examples, the structural formula of the iodonium salt-based compound can be represented by the following formula (8).

[Formula (8)] $[(R1)_2I]^+ [A]^-$

(In the formula, $[(R1)_2I]^+$ is a cation part, $[A]^-$ is an anion part, R1 shown in the formula (8) represents an organic group bonded to I, and R1 may be the same or different. R1 represents, for example, an aryl group having 6 to 30 carbon atoms, a heterocyclic group having 4 to 30 carbon atoms, an alkyl group having 1 to 30 carbon atoms, an alkenyl group having 2 to 30 carbon atoms, or an alkynyl group having 2 to 30 carbon atoms, which may have at least one substituted group selected from the group consisting of groups such as alkyl, hydroxy, alkoxy, alkylcarbonyl, arylcarbonyl, alkoxycarbonyl, aryloxycarbonyl, arylthiocarbonyl, acyloxy, arylthio, alkylthio, aryl, heterocycle, aryloxy, alkylsulfinyl, arylsulfinyl, alkylsulfonyl, arylsulfonyl, alkyleneoxy, amino, cyano, nitro groups and halogens.)

In the above, examples of the aryl group having 6 to 30 carbon atoms include a monocyclic aryl group such as a phenyl group and a condensed polycyclic aryl group such as a naphthyl, anthrasenyl, phenanthrenyl, pyrenyl, chrysenyl, naphthacenyl, benzanthrasenyl, anthraquinolyl, fluorenyl, naphthoquinone and anthraquinone.

Examples of the heterocyclic group having 4 to 30 carbon atoms include cyclic groups containing 1 to 3 heteroatoms such as oxygen, nitrogen, and sulfur, which may be the same or different. Specific examples include a monocyclic heterocyclic group such as thienyl, furanyl, pyranyl, pyrrolyl, oxazolyl, thiazolyl, pyridyl, pyrimidyl and pyrazinyl, and a condensed polycyclic heterocyclic group such as indolyl, benzofuranyl, isobenzofuranyl, benzothienyl, isobenzothienyl, quinolyl, isoquinolyl, quinoxalinyl, quinazolinyl, carbazolyl, acridinyl, phenothiazinyl, phenazinyl, xanthenyl, thianthrenyl, phenoxazinyl, phenoxathiinyl, chromanyl, isochromanyl, dibenzothienyl, xanthonyl, thioxanthonyl and dibenzofuranyl.

Specific examples of the alkyl groups having 1 to 30 carbon atoms include a linear alkyl group such as methyl, ethyl, propyl, butyl, hexadecyl and octadecyl, a branched alkyl group such as isopropyl, isobutyl, sec-butyl, tert-butyl, isopentyl, neopentyl, tert-pentyl, isohexyl and a cycloalkyl groups such as cyclopropyl, cyclobutyl, cyclopentyl and cyclohexyl.

In addition, specific examples of the alkenyl group having 2 to 30 carbon atoms include a linear chain or branched group such as vinyl, allyl, 1-propenyl, isopropenyl, 1-butenyl, 2-butenyl, 3-butenyl and 1-methyl-1-propenyl.

Further, specific examples of the alkynyl group having 2 to 30 carbon atoms include a linear chain or branched group such as ethynyl, 1-propynyl, 2-propynyl, 1-butynyl, 2-butynyl, 3-butynyl, 1-methyl-1-propynyl and 1-methyl-2-propynyl.

The above-described aryl group having 6 to 30 carbon atoms, heterocyclic group having 4 to 30 carbon atoms, alkyl group having 1 to 30 carbon atoms, alkenyl group having 2 to 30 carbon atoms and alkynyl group having 2 to 30 carbon atoms may have at least one substituted group. Specific examples of the substituted group include a linear alkyl group having 1 to 18 carbon atoms such as methyl, ethyl, propyl, butyl and octadecyl; a branched alkyl group having 1 to 18 carbon atoms such as isopropyl, isobutyl, sec-butyl and tert- butyl; a cycloalkyl group having 3 to 18 carbon atoms such as cyclopropyl, cyclobutyl, cyclopentyl and cyclohexyl; a hydroxy group; a linear chain or branched alkoxy group having 1 to 18 carbon atoms such as methoxy, ethoxy, propoxy, isopropoxy, butoxy, isobutoxy, sec-butoxy, tert-butoxy and dodecyloxy; a linear chain or branched alkylcarbonyl group having 2 to 18 carbon atoms such as acetyl, propionyl, butanoyl, 2-methylpropionyl, heptanoyl, 2-methylbutanoyl, 3-methylbutanoyl and octanoyl; an arylcarbonyl group having 7 to 11 carbon atoms such as benzoyl and naphthoyl; a linear chain or branched alkoxycarbonyl group having 2 to 19 carbon atoms such as methoxycarbonyl, ethoxycarbonyl, propoxycarbonyl, isopropoxycarbonyl, butoxycarbonyl, isobutoxycarbonyl, sec-butoxycarbonyl and tert-butoxycarbonyl; an aryloxycarbonyl group having 7 to 11 carbon atoms such as phenoxycarbonyl and naphthoxycarbonyl; an arylthiocarbonyl group having 7 to 11 carbon atoms such as phenylthiocarbonyl and naphthoxythiocarbonyl; a linear chain or branched acyloxy group having 2 to 19 carbon atoms such as acetoxy, ethylcarbonyloxy, propylcarbonyloxy, isobutylcarbonyloxy, sec-butylcarbonyloxy, tert-butylcarbonyloxy and octadecylcarbonyloxy; an arylthio group having 6 to 20 carbon atoms such as phenylthio, biphenylthio, methylphenylthio, chlorophenylthio, bromophenylthio, fluorophenylthio, hydroxyphenylthio, methoxyphenylthio, naphthylthio, 4-[4-(phenylthio) benzoyl] phenylthio, 4-[4-(phenylthio) phenoxy] phenylthio, 4-[4-(phenylthio) phenyl] phenylthio, 4-(phenylthio) phenylthio, 4-benzoyl phenylthio, 4-benzoyl-chlorophenylthio, 4-benzoyl-methylthio phenylthio, 4-(methylthiobenzoyl) phenylthio and 4-(p-tert-butylbenzoyl) phenylthio; a linear chain or branched alkylthio group having 1 to 18 carbon atoms such as methylthio, ethylthio, propylthio, tert-butylthio, neopentylthio and dodecylthio; an aryl group having 6 to 10 carbon atoms such as phenyl, tolyl, dimethylphenyl and naphthyl; a heterocycle group having 4 to 20 carbon atoms such as thienyl, furanyl, pyranyl, xanthenyl, chromanyl, isochromanyl, xanthonyl, thioxanthonyl and dibenzofuranyl; an aryloxy group having 6 to 10 carbon atoms such as phenoxy and naphthyloxy; a linear chain or branched alkylsulfinyl group having 1 to 18 carbon atoms such as methylsulfinyl, ethylsulfinyl, propylsulfinyl, tert-pentylsulfinyl and octylsulfinyl; an arylsulfinyl group having 6 to 10 carbon atoms such as phenylsulfinyl, tolylsulfinyl and naphthylsulfinyl; a linear chain or branched alkylsulfonyl group having 1 to 18 carbon atoms such as methylsulfonyl, ethylsulfonyl, propylsulfonyl, isopropylsulfonyl, butylsulfonyl and octylsulfonyl; an arylsulfonyl group having 6 to 10 carbon atoms such as phenylsulfonyl, tolylsulfonyl (tosyl), naphthylsulfonyl; an alkyleneoxy groups; a cyano groups; a nitro groups; and halogens such as fluorine, chlorine, bromine and iodine.

Among the iodonium salt-based compounds, the aryl iodonium salt is preferable because of having high stability. Further, it is preferable that the aryl group has a substituent in order to improve fat solubility. Specifically, a linear alkyl group such as methyl, propyl, octyl, decyl, undecyl, dodecyl and tridecyl, a branched alkyl group such as isopropyl, isobutyl, sec-butyl, tert-butyl, isopentyl, neopentyl, tert-pentyl and isohexyl, a functional group in which one or more ofH of these is substituted with F, a perfluoroalkyl group and halogen is suitable as the substituent.

The structure of an anion portion of the iodonium salt-based compound is not particularly limited, and examples include those having atoms such as halogen, P, S, B, Al and Ga. From the viewpoint of safety, anions having As or Sb can be used, but they are not preferable in dental applications. Further, the anion preferably has an organic group such as an alkyl group and/or an alkoxy group and/or an aryl group, and further, most preferably has an organic group such as an alkyl group and/or an alkoxy group and/or an aryl group, in which at least one H is substituted with F. Since the iodonium salt-based compound having such an anion has high solubility with respect to the dental composite resin, it is expected to preventing precipitation during low-temperature storage or long-term storage and to shorten the time for preparing due to dissolving in the composition in a short time. Further, an iodonium salt-based compound of an anion having an organic group such as an alkyl group and/or an alkoxy group and/or an aryl group, in which at least one H is substituted with F can be expected to have higher solubility. When the photoacid generator is precipitated, there is a case that it may cause a decrease in light color stability and a decrease in bending strength, and therefore it is not preferable. As the anion having an organic group such as an alkyl group and/or an alkoxy group and/or an aryl group, in which at least one H is substituted with F, an anion having any atom can be used. However, from the viewpoint of versatility and safety, those having one or more of P, S, B, Al and Ga are preferable.

Examples of the anion having no alkyl group and/or alkoxy group and/or aryl group include halogens such as chloride and bromide, perhalonic acids such as perchloric acid, aromatic sulfonic acids such as p-toluenesulfonate, camphorsulfonnic acids, nitrates, acetates, chloroacetates, carboxylates, phenolates, tetrafluoroborates, hexafluorophosphates, hexafluoroantimonates, hexafluoroarsenates and the like. Among these, p-toluenesulfonate, camphorsulfonic acid and carboxylate are preferably used.

Since the anionic part of $[A]^-$ of the iodonium salt-based compound of the formula (8) improves the solubility to the dental composite resin, it is preferable that the anion has an organic group such as alkyl group and/or alkoxy group and/or aryl group, in which at least one H is substituted with F. Specifically, the number of carbon atoms of the alkyl group in the

anion part of $[A]^-$ of the iodonium salt-based compound of the formula (8) is 1 to 8, and preferably 1 to 4. Specific examples include a linear alkyl group such as methyl, ethyl, propyl, butyl, pentyl and octyl, a branched alkyl group such as isopropyl, isobutyl, sec-butyl and tert-butyl, and a cycloalkyl group such as cyclopropyl, cyclobutyl, cyclopentyl and cyclohexyl. The ratio (F/H) of the number of hydrogen atoms to fluorine atoms in the alkyl group is 4 or more, and the ratio (F/H) of the number of hydrogen atoms to fluorine atoms in the alkyl group is preferably 9 or more. More preferably, all hydrogen atoms of the hydrocarbon are substituted with fluorine. An iodonium salt consisting of an anion having an alkyl group having a different ratio of a hydrogen atom and a fluorine atom may be compounded in the dental composite resin.

Further, specific examples of the alkyl group include a linear chain or branched perfluoroalkyl group such as $CF_3$, $CF_3CF_2$, $(CF_3)_2CF$, $CF_3CF_2CF_2$, $CF_3CF_2CF_2CF_2$, $(CF_3)_2CFCF_2$, $CF_3CF_2(CF_3)CF$ and $(CF_3)_3C$.

The number of carbon atoms of the alkyl group in the anion part of $[A]^-$ of the iodonium salt-based compound of the formula (8) is 1 to 8, and preferably 1 to 4. Specific examples include a linear alkoxy group such as methoxy, ethoxy, propoxy, butoxy, pentoxy and octoxy, and a branched alkoxy group such as isopropoxy, isobutoxy, sec-butoxy and tert-butoxy. The ratio (F/H) of the number of hydrogen atoms to fluorine atoms in the alkyl group is 4 or more, and the ratio (F/H) of the number of hydrogen atoms to fluorine atoms in the alkyl group is preferably 9 or more. More preferably, all hydrogen atoms of the hydrocarbon are substituted with fluorine. An iodonium salt consisting of an anion having an alkoxy group having a different ratio of a hydrogen atom and a fluorine atom may be compounded in the dental composite resin.

Further, specific examples of the alkoxy group include a linear or branched perfluoroalkoxy group such as $CF_3O$, $CF_3CF_2O$, $CF_3CF_2CF_2O$, $(CF_3)_2CFO$, $CF_3CF_2CF_2CF_2O$, $(CF_3)_2CFCF_2O$, $CF_3CF_2(CF_3)CFO$, $CF_3CF_2CF_2CF_2CF_2O$ and $CF_3CF_2CF_2CF_2CF_2CF_2CF_2CF_2O$.

The phenyl group in the anion part of $[A]^-$ of the iodonium salt compound of the formula (8) may be a phenyl group, in which at least one H is substituted with fluorine atom, an alkyl group and/or an alkoxy group substituted with fluorine atom. The alkyl group and/or alkoxy group substituted with fluorine atom are preferably those described above. Specific examples of particularly preferable phenyl group include perfluorophenyl group such as pentafluorophenyl group ($C_6F_5$), trifluorophenyl group ($C_6H_2F_3$), tetrafluorophenyl group ($C_6HF_4$), trifluoromethylphenyl group ($CF_3C_6H_4$), bis (trifluoromethyl) phenyl group ($(CF_3)_2C_6H_3$), pentafluoroethyl phenyl group ($CF_3CF_2C_6H_4$), bis (pentafluoroethyl) phenyl group ($(CF_3)_2C_6H_3$), trifluoromethyl fluorophenyl group ($CF_3C_6H_3F$), bistrifluoromethyl fluorophenyl group ($(CF_3)_2C_6H_2F$), pentafluoroethyl fluorophenyl group ($CF_3CF_2C_6H_3F$), bispentafluoroethyl fluorophenyl group $(CF_3CF_2)_2C_6H_2F$ and the like. An iodonium salt consisting of an anion having a phenyl group having a different ratio of a hydrogen atom and a fluorine atom may be compounded in the dental composite resin.

As specific examples of the anion portion of $[A]^-$ of the iodonium salt compound of the formula (8), examples of the anion having P include $[(CF_3CF_2)_3PF_3]^-$, $[(CF_3CF_2CF_2)_3PF_3]^-$, $[((CF_3)_2CF)_2PF_4]^-$, $[((CF_3)_2CF)_3PF_3]^-$, $[((CF_3)_2CF)_4PF_2]^-$, $[((CF_3)_2CFCF_2)_2PF_4]^-$, $[((CF_3)_2CFCF_2)_3PF_3]^-$ and the like. Examples of the anion having S include $[(CF_3SO_2)_3C]^-$, $[(CF_3CF_2SO_2)_3C]^-$, $[(CF_3CF_2CF_2SO_2)_3C]^-$, $[(CF_3CF_2CF_2CF_2SO_2)_3C]^-$, $[CF_3CF_2CF_2CF_2SO_3]^-$, $[CF_3CF_2CF_2SO_3]^-$, $[(CF_3CF_2SO_2)_3C]^-$, $[(SO_2CF_3)_3N]^-$, $[(SO_2CF_2CF_3)_2N]^-$, $[((CF_3)C_6H_4)SO_3]^-$, $[SO_3(CF_2CF_2CF_2CF_2)SO_3]^{2-}$ and the like. Examples of the anion having B include $[B(C_6F_5)4]^-$, $[(C_6H_5)B((CF_3)_2C_6H_3)_3]^-$, $[(C_6H_5)B(C_6F_5)_3]^-$ and the like. Examples of an anion having Ga include $[((CF_3)_4Ga)]^-$, $[Ga\ (C_6F_5)_4]^-$ and the like. Examples of anions having Al include $[((CF_3)_3CO)_4Al]^-$, $[((CF_3CF_2)_3CO)_4Al]^-$.

In the case that the dental composite resin of the present invention contains the (C) photoacid generator, the compounding amount is preferably 0.1 to 5 parts by mass, more preferably 0.2 to 5 parts by mass, with respect to 100 parts by mass of the total amount of the (A) polymerizable monomer. When the compounding amount of the photoacid generator is less than 0.1 parts by mass, there is a case that the expected polymerization promoting ability is not exhibited and the curing becomes insufficient. When the compounding amount is more than 5 parts by mass, although sufficient curability is exhibited, there is a case that the sensitivity to light is lowered to shorten operation time, and discoloration such as browning of the cured body increases.

The photoacid generator that can be used in the dental composite resin of the present invention is not limited to the photoacid generator described in the specific example, and two or more types can be used in combination.

The dental composite resin of the present invention may comprise only an aryl iodonium salt which is a salt of an anion having an organic group and one or more atoms of P, B, Al, S, and Ga, and an aryl iodonium cation as the (C) photoacid generator. The dental composite resin of the present invention may comprise only an aryl iodonium salt which is a salt of an anion having an organic group in which at least one H may be substituted with F and one or more atoms of P, B, Al, S, and Ga, and an aryl iodonium cation as the (C) photoacid generator.

[(D) Photopolymerization Accelerator]

The (D) photopolymerization accelerator which is used for the dental composite resin of the present invention is not particularly limited as long as it has polymerization promoting ability, and any known photopolymerization accelerator commonly used in the dental field may be used without any limitation. As the (D) photopolymerization accelerator, a primary to tertiary amine compound such as an aromatic amine compound and an aliphatic amine compound, an organic

metal compound, a phosphine compound, and the like can be used. Among these, in the present invention, it is possible to contain (D1) aliphatic tertiary amine compound. In addition, an organic metal compound may be included because of its good light color stability.

Aromatic amine compound refers to a compound in which one or more H of ammonia ($NH_3$) is replaced with an aromatic ring. Aromatic amine compound in which one H of $NH_3$ is substituted with an aromatic ring is classified into an aromatic primary amine compound, aromatic amine compound in which one H of $NH_3$ is substituted with an aromatic ring and one H of remaining two H is substituted with an aromatic ring or an alkyl group is classified into an aromatic secondary amine compound, and aromatic amine compound in which one H of $NH_3$ is substituted with an aromatic ring and remaining two H are substituted with an aromatic ring or an alkyl group is classified into an aromatic tertiary amine compound.

Specific examples of the aromatic primary amine compound include aniline. Specific examples of the aromatic secondary amine compound include N-protected amino acid (ester) such as N-phenyl benzylamine, N-benzyl-p-anisidine, N-benzyl-o-phenetidine, N-phenylglycine ethyl and N-phenylglycine. Specific examples of the aromatic tertiary amine compound include N,N-dimethylaniline, N,N-diethylaniline, N,N-di-n-butylaniline, N,N-dibenzylaniline, p-N,N-dimethyl-toluidine, m-N,N-dimethyl-toluidine, p-N,N-diethyl-toluidine, p-bromo-N,N-dimethylaniline, m-chloro-N,N-dimethylaniline, p-dimethylamino benzaldehyde, p-dimethylamino acetophenone, p-dimethylamino benzoic acid, p-dimethylamino benzoic acid ethyl ester, p-dimethylamino benzoic acid isoamyl estel, p-dimethylamino benzoic acid 2-butoxyethyl, p-dimethylamino benzoic acid 2-ethylhexyl, p-dimethylamino benzoic acid amino ester, N,N-dimethyl anthranic acid methyl ester, N,N-dihydroxyethyl aniline, N,N-diisopropanol aniline, p-N,N-dihydroxyethyl-toluidine, p-N,N-dihydroxypropyl-toluidine, p-dimethylamino phenyl alcohol, p-dimethylamino styrene, N,N-dimethyl-3,5-xylidine, 4-dimethylamino pyridine, N,N-dimethyl-$\alpha$-naphthylamine, N,N-dimethyl-$\beta$-naphthylamine and the like. Among them, p-dimethylamino benzoic acid ethyl ester is preferable.

Specific examples of the above organic metal compound include an organic metal compound containing scandium (Sc), titanium (Ti), vanadium (V), manganese (Mn), iron (Fe), cobalt (Co), nickel (Ni), copper (Cu), tin (Sn), zinc (Zn) and/or zirconia (Zr), and an organic metal compound containing tin (Sn), vanadium (V) and/or copper (Cu) is preferable. Specific examples of the organic metal compound containing tin (Sn) include dibutyl-tin-diacetate, dibutyl-tin-dimaleate, dioctyl-tin-dimaleate, dioctyl-tin-dilaurate, dibutyl-tin-dilaurate, dioctyl-tin- dinonanoate, dioctyl-tin-S,S'-bis-isooctyl mercapto acetate, tetramethyl-1,3-diacetoxy distanoxane and the like. Specific examples of the organic metal compound containing vanadyl (V) include acetylacetone vanadium, divanadium tetraoxide, vanadyl acetylacetonate, vanadyl stearate oxide, vanadyl oxalate, vanadyl sulphate, oxobis (1-phenyl-1,3-butandionate) vanadium, bis (maltlate) oxovanadium, vanadium pentoxide and sodium metavanadate. Specific examples of the organic metal compound containing copper (Cu) include copper acetylacetone, copper naphthenate, copper octylate, copper stearate and copper acetate.

The phosphine compound refers to a compound which is trisubstituted on P atom with organic groups, and the aromatic phosphine compound refers to a compound which is substituted on P atom with a phenyl group which may have one or more substituents. Specific examples of the phosphine compound include trimethylphosphine, tributylphosphine, trihexylphosphine, tri-n-octylphosphine, tricyclohexylphosphine, tri (2-thienyl) phosphine, diphenylpropyl phosphine, di-tert-butyl (3-methyl-2-butenyl) phosphine, methyldiphenyl phosphine, triphenyl phosphine, 2-(diphenylphosphino) styrene, 3-(diphenylphosphino) styrene, 4-(diphenylphosphino) styrene, allyldiphenyl phosphine, 2-(diphenylphosphino) benzaldehyde, 3-(diphenylphosphino) benzaldehyde, 4-(diphenylphosphino) benzaldehyde, 2-(phenylphosphine) benzoic acid, 3-(phenylphosphino) benzoic acid, 4-(phenylphosphino) benzoic acid, tris (2-methoxyphenyl) phosphine, tris (3-methoxyphenyl) phosphine, tris (4-methoxyphenyl) phosphine, 2-(diphenylphosphino) biphenyl, tris (4-fluorophenyl) phosphine, tri (o-trill) phosphine, tri (m-trill) phosphine, tri (p-trill) phosphine, 2-(dimethylamino) phenyldiphenyl phosphine, 3-(dimethylamino) phenyldiphenyl phosphine, 4-(dimethylamino) phenyldiphenyl phosphine, 2,2'-bis (diphenylphosphino) biphenyl, bis [2-(diphenylphosphino) phenyl] ether and the like. Among them, triphenylphosphine, 4-(phenylphosphino) benzoic acid, tri (o-tolyl) phosphine, tri (m-tolyl) phosphine and tri (p-tolyl) phosphine are preferable.

Aliphatic amine compounds refer to compounds in which one or more H of ammonia ($NH_3$) are substituted with alkyl group. As for the alkyl group, $CH_3$- and -$CH_2$- are classified as a primary alkyl group, the one in which one H of -$CH_2$- is substituted with a substituent is classified as a secondary alkyl group, and the one in which two H of -$CH_2$- are substituted with substituents is classified as a tertiary alkyl group. Aliphatic amine in which one H of $NH_3$ is substituted with an alkyl group is classified into an aliphatic primary amine compound, aliphatic amine compound in which two H of $NH_3$ are substituted with an alkyl group is classified into an aliphatic secondary amine compound, and aliphatic amine compound in which three H of $NH_3$ are substituted with an alkyl group is classified into an aliphatic tertiary amine compound.

Specific examples of the aliphatic primary amine compound include amino acid or amino acid ester such as benzhydrylamine, triphenylmethylamine and glycine. Specific examples of the aliphatic secondary amine compound include dibenzylamine, N-benzyl-1-phenylethylamine, bis (1-phenylethyl) amine, bis (4-cyanobenzyl) amine, N-benzyl protected amino acid and N-benzyl protected amino acid ester. Specific examples of the aliphatic tertiary amine compound include tributylamine, tripropylamine, triethylamine, N,N-dimethyl hexylamine, N,N-dimethyl dodecylamine, N,N-dimethyl stearylamine, N-[3-(dimethylamino) propyl] acrylamide, N,N-dimethyl formamide dimethylacetal, N,N-dimethylacetamide

dimethylacetal, N,N-dimethylformamide diethylacetal, N,N-dimethylformamide dipropylacetal, N,N-dimethylformamide di-tert-butylacetal, 1-(2-hydroxyethyl) ethyleneimine, N,N-dimethyl ethanolamine, N,N-dimethyl isopropanolamine, N,N-diisopropyl ethanolamine, N-methyl diethanolamine, N-ethyl diethanolamine, N-ethyl diethanolamine, N-butyl diethanolamine, N-lauryl diethanolamine, N-stearyl diethanolamine, triethanolamine, triisopropanolamine, tribenzylamine, dibenzylglycine ethylester, N'-(2-hydroxyethyl)-N,N,N'-trimethylethylene diamine, 2-(dimethylamino)-2-methyl-1-propanol, N,N-dimethyl-2,3-dihydroxy propylamine, N,N-diethylethanolamine, 1-methyl-3-pyrrolidinol, 1-(2-hydroxyethyl) pyrrolidine, 1-isopropyl-3-pyrrolidinol, 1-piperidin ethanol, 2-[2-(dimethylamino) ethoxy] ethanol, N,N-dimethylglycine, N,N-dimethylglycine methyl, N,N-diethylglycine methyl, N,N-dimethylglycine ethyl, N,N-diethylglycine sodium, 2-(dimethylamino) ethylacetate, N-methylimimino diacetic acid, N,N-dimethylamino ethylacrylate, N,N-diethylamino ethylmethacrylate, N,N-diisopropylamino ethylmethacrylate, N,N-dibutylamino ethylmethacrylate, N,N-dibenzylamino ethylmethacrylate, 3-dimethylamino propionitrile, tris (2-cyanoethyl) amine, N,N-dimethyl allylamine, N,N-diethyl allylamine, triallylamine and pentamethylpiperidyl methacrylate.

The dental composite resin of the present invention may contain (D1) aliphatic tertiary amine compound as the (D) photopolymerization accelerator. The (D1) aliphatic tertiary amine compound, serves as a photopolymerization initiator suitable for dental applications by combining with the (B) photosensitizer and the (C) photoacid generator. Examples of the (D1) aliphatic tertiary amine compound include those described above. Preferable examples include N,N-dimethylamino ethylacrylate, N,N-diethylamino ethylmethacrylate, N,N-diisopropylamino ethylmethacrylate, N,N-dibutylamino ethylmethacrylate, N,N-dibenzylamino ethylmethacrylate, pentamethylpiperidyl methacrylate, triisopropanolamine, tribenzylamine, dibenzylglycine ethyl ester, and dibenzylpropanolamine. Among them , pentamethylpiperidyl methacrylate, tribenzylamine, and dibenzylglycine ethyl ester are preferable.

The dental composite resin of the present invention may contain an aromatic tertiary amine compound as the (D) polymerization accelerator. However, in the case of containing an aromatic tertiary amine compound, there is a case where the cured dental composite resin discolors upon exposure to light and color stability is poor. Therefore, in the case of containing an aromatic tertiary amine compound, it is preferable to contain an ultraviolet absorber. Furthermore, since the dental composite resin of the present invention can exhibit good mechanical property in the case of not containing an aromatic tertiary amine compound as the (D) polymerization accelerator, it is possible to not contain an aromatic tertiary amine compound.

The type of the (D) photopolymerization accelerator may be appropriately selected according to the type and the compounding amount of other components to be combined. In addition, the (D) photopolymerization accelerator may be used not only singly but also in combinations of two or more.

The compounding amount of the (D) photopolymerization accelerator is preferably 0.2 to 10 parts by mass, more preferably 0.5 to 10 parts by mass, with respect to 100 parts by mass of the total amount of the (A) polymerizable monomer contained in the dental composite resin. When the compounding amount of the (D) photopolymerization accelerator is less than 0.2 parts by mass, the polymerization promoting ability is poor and the curing tends to be insufficient. When the compounding amount is more than 10 parts by mass, although sufficient curability is exhibited, there is a case that the environmental light stability is shortened, and discoloration of the cured body increases.

The compounding amount of the (D1) aliphatic tertiary amine compound with respect to 100 parts by mass of the total amount of the (A) polymerizable monomer contained in the dental composite resin is preferably 0.2 to 5 parts by mass, more preferably 0.5 to 5 parts by mass. When the compounding amount of the (D1) aliphatic tertiary amine compound is less than 0.2 parts by mass, the polymerization promoting ability is poor and the curing tends to be insufficient. When the compounding amount is more than 5 parts by mass, although sufficient curability is exhibited, there is a case that the environmental light stability is shortened, and discoloration of the cured body increases.

There is no problem even if these (B) photosensitizers, (C) photoacid generators and (D) photopolymerization accelerators, which are polymerization initiators, are subjected to a secondary treatment such as finely pulverization, adsorption on a carrier and encapsulation in a microcapsule, if necessary. Furthermore, these photo polymerization initiators can be used not only singly but also in combinations of two or more, regardless of the polymerization manner or the polymerization method.

[(E) Filler]

As the (E) filler used in the present invention, a known filler commonly used can be used without any limitation.

The type of the (E) filler is not limited as long as it is a known filler, and a filler suitable for the application can be compounded, and it is preferable that a filler such as an inorganic filler, an organic filler, an organic-inorganic composite filler and an ion sustained release glass is compounded. In the dental composite resin of the present invention, the filler described in the specific example may be used alone, or two or more kinds of fillers may be used in combination.

As the inorganic filler, the chemical composition is not particularly limited, but specific examples include silicon dioxide, alumina, titania, silica-titania, silica-titania-barium oxide, silica-zirconia, silica-alumina, lanthanum glass, borosilicate glass, soda glass, barium glass, strontium glass, glass ceramic, aluminosilicate glass, barium boroaluminosilicate glass, strontium boroaluminosilicate glass, fluoro boroaluminosilicate glass fluoroaluminosilicate glass, calcium fluoroaluminosilicate glass, strontium fluoroaluminosilicate glass, barium fluoroaluminosilicate glass, strontium calcium fluoroaluminosilicate glass and the like. Particularly, barium fluoroaluminosilicate glass, strontium fluoroaluminosilicate glass, fluoroaluminosilicate glass and the like, which are used in dental glass ionomer cement, resin reinforced glass ionomer cement and resin cement and the like, can also be suitably used. The fluoroaluminosilicate glass as used herein has a basic structure of silicon oxide and aluminum oxide and contains an alkali metal for introducing non-crosslinked oxygen. The fluoroaluminosilicate glass further has an alkaline earth metal including strontium and fluorine as modified/coordinated ions. The fluoroaluminosilicate glass may be also a composition in which a lanthanoid series element is incorporated into the skeleton in order to impart further radiopacity. This lanthanoid series element also participates in the composition as a modified/coordinated ion.

Specific examples of the organic filler include polymers such as polymethyl methacrylate, polyethyl methacrylate, methyl methacrylate-ethyl methacrylate copolymer, ethyl methacrylate-butyl methacrylate copolymer, methyl methacrylate-trimethylolpropane methacrylate copolymer, polyvinylchloride, polystyrene, chlorinated polyethylene, nylon, polysulfone, polyethersulfone and polycarbonate.

Specific examples of the organic/inorganic composite filler include one obtained by covering the surface of a filler with a polymerizable monomer by polymerization, one obtained by mixing a filler and a polymerization monomer and polymerizing the monomer and thereafter grinding the resultant to a proper particle size, one obtained by dispersing a filler in a polymerizable monomer in advance for emulsion polymerization or suspension polymerization, one obtained by spray-drying a polymerizable monomer and a solvent which are dispersed with a filler in advance and polymerizing, and one obtained by spray-drying a solvent which is dispersed with a filler in advance, impregnating a polymerizable monomer and polymerizing, but are not limited thereto at all.

The feature of the ion sustained release glass is that at least one of fluorine ion, strontium ion, borate ion and aluminum ion is sustained release. Among these ions, it is preferable that a plurality of these ions are released at the same time.

As the ion sustained release glass used in the present invention, any ion sustained release glass can be used without any limitation as long as such ion sustained release glass includes one or more kinds of glass skeleton forming elements which form a glass skeleton, and one or more kinds of glass modifying elements which modify the glass skeleton. These ion sustained release glasses may be used alone or in combination of two or more thereof. Further, in the present invention, glass amphoteric elements that play a role of either a glass skeleton forming element or a glass modifying element depending on the glass composition are included within a category of a glass skeleton forming element. Specific examples of the glass skeleton forming element contained in the ion sustained release glass, include silica, aluminum, boron and phosphorus, and these can be used not only singly but also in combinations of a plurality thereof. Specific examples of the glass modifying element include halogen elements such as fluorine, bromine and iodine, alkali metal elements such as sodium and lithium, and alkali earth metal elements such as calcium and strontium, and these can be used not only singly but also in combinations of a plurality thereof. Among them, a glass composition including silica, aluminum or boron as the glass skeleton forming element and including fluorine, sodium or strontium as the glass modifying element is preferable, and specific examples include a silica glass, a fluoroaluminosilicate glass, a fluoroborosilicate glass and a fluoroalumino borosilicate glass containing strontium or sodium. Furthermore, from the viewpoint of sustainably releasing fluorine ion, strontium ion, borate ion or aluminum ion, a fluoroalumino borosilicate glass containing strontium is more preferable. Example of the glass composition range thereof is as follows: $SiO_2$: 15 to 35% by mass, $Al_2O_3$: 15 to 30% by mass, $B_2O_3$: 5 to 20% by mass, SrO: 20 to 45% by mass, F: 5 to 15% by mass, and $Na_2O$: 0 to 10% by mass. The glass composition can be confirmed by using an instrumental analysis such as an elementary analysis, Raman spectrum, and fluorescence X-ray analysis, and there is no problem as long as the actual measurement by any analysis methods matches to these composition ranges.

A method for preparing an ion sustained release glass is not particularly limited, and the glass can be produced by a preparing method such as a melting method or a sol-gel method. Among them, a preparing method by a melting method using a melting furnace is preferable from the viewpoint of ease of design of the glass composition, including raw material selection. The ion sustained release glass used in the present invention has an amorphous structure, but there is no problem even if it contains a partially crystalline structure, and further, a mixture of a glass having an amorphous structure and a glass having a crystal structure may be used without any problem. Whether the structure of the glass is an amorphous structure can be confirmed using an X-ray diffraction analysis or an analysis instrument such as a transmission electron microscope. In particular, the ion sustained release glass used in the present invention sustainably releases various ions by means of an equilibrium relation with ion concentrations in the external environment, and therefore an amorphous structure which is a homogeneous structure is preferable.

Further, in order to enhance ion sustained release property of the ion sustained release glass, it is a preferable embodiment to functionalize the glass surface by a surface treatment, thereby improving the ion sustained-release property. Specific examples of a surface treatment material used in the surface treatment include a surfactant, a fatty acid, an organic acid, an inorganic acid, a monomer, a polymer, various coupling materials, a silane compound, a metal alkoxide

compound, and a partially condensed product thereof. Among these surface treatment materials, it is preferable to perform composite surface treatment using an acidic polymer and a silane compound.

The composite surface treatment is a method for surface treatment by using an acidic polymer after coating the surface of the ion sustained release glass with a silane compound. Specific example is described in the following. A silane compound represented by the formula (9) is mixed in an aqueous dispersion containing an ion sustained release glass finely pulverized to a desired average particle diameter (D50) by pulverization or the like. The mixture is hydrolyzed or partially hydrolyzed in the system to prepare a silanol compound. Then the silanol compound is condensed to form a polysiloxane, and then the surface of the ion sustained release glass is coated with the polysiloxane to obtain a polysiloxane coated ion sustained release glass.

[Formula (9)]

[Chemical formula 1]

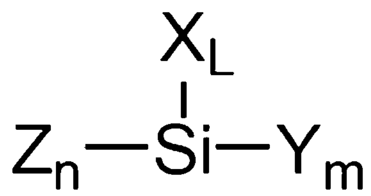

(in the formula, Z is RO-, X is halogen, Y is OH-, R is an organic group whose carbon number is less than or equal to 8, and n, m, and L are each an integer from 0 to 4 where n + m + L = 4)

Specific Examples of the silane compound represented by the formula (9) include tetramethoxysilane, tetraethoxysilane, tetrapropoxysilane, tetraallyloxysilane, tetrabutoxysilane, tetrakis (2-ethylhexyloxy) silane, trimethoxychlorosilane, triethoxychlorosilane, triisopropoxychlorosilane, trimethoxyhydroxysilane, diethoxydichlorosilane, tetraphenoxysilane, tetrachlorosilane and silicon hydroxide (silicon oxide hydrate), more preferable include tetramethoxysilane and tetraethoxysilane.

A low condensed product of the silane compound represented by the formula (9) is more preferable. Examples include a low condensed silane compound obtained by partial hydrolysis and condensation of tetramethoxysilane and tetraethoxysilane. These compounds can be used singly or in combination. An organosilane compound can also be added in the polysiloxane treatment as a part of the silane compound represented by the formula (9).

The polysiloxane coated ion sustained release glass obtained in the above step is subjected to an acidic polymer treatment for reacting with an acidic polymer to provide a most preferable ion sustained release glass. The acidic polymer treatment can be conducted by using an equipment commonly used in the art as long as the equipment is a dry fluid type stirring machine, and examples include a Henschel mixer, a super mixer and a high-speed mixer. The reaction of the ion sustained release glass formed with the polysiloxane film and an acidic polymer can be conducted by contacting an acidic polymer solution by impregnating, spraying or the like. As an example, it is only necessary to perform a dry fluid of the polysiloxane coated ion sustained release glass, dispersing the acid polymer solution from above in the flow state, and sufficiently stirring. The method for dispersing the acidic polymer solution is not particularly limited, and a dropping or spraying method that can uniformly disperse is more preferable. The reaction is preferably conducted around room temperature, and when the temperature increases, there is a case where the reaction of an acid reactive element and the acidic polymer is fast, and thereby formation of a cement phase is not uniform.

It is preferable to remove the water content in the cement reaction phase by performing a heat treatment after the reaction. If water content remains in the cement reaction phase, it is disadvantageous in terms of strength, but since the filler of the present invention is covered and strengthened by the coupling agent condensate film, the decrease in mechanical strength is suppressed. The heat treatment method after the acid polymer treatment is not particularly limited, and can be performed by a known general method. As the equipment used for the heat treatment, a box-type hot air dryer or the like, a rotary heat treatment device capable of uniform heating and the like are preferable. The heat treatment temperature is in the range from room temperature to 200 °C, more preferably in the range from 40 to 150 °C. When the temperature is lower than this range, there is a risk that the removal of the aqueous medium is insufficient, and when the temperature is higher than this range, there is a risk that the organic layer of the acidic polymer is decomposed or discolored. Since the heat treatment period depends on the capacity of the dryer and the like, there is no problem as long as the aqueous medium can be sufficiently removed. After a heat treatment, a heat-treated product can be easily deagglomerated by application of a shear force or an impact force, and the deagglomerating method can be performed by, for example, the equipment used in the above reaction.

A solvent used for preparing the acid polymer solution used for the reaction may be solvent which dissolves the acid polymer without any problems. Examples of the solvent include water, ethanol, and acetone. Of these, water is

particularly preferable. When water is used, an acid group of the acid polymer dissociates and reacts uniformly with the surface of polysiloxane-coated ion sustained release glass. The weight average molecular weight of the polymer dissolved in the acid polymer solution is in the range of 2000 to 50000, and preferably in the range of 5000 to 40000. In the case of treating with the acidic polymer having a weight average molecular weight of less than 2000, there is a tendency that an acidic polymer reaction phase is not formed in the ion sustained release glass, and as a result, the ion sustained release property is low. On the other hand, in the case of treating with an acidic polymer having a weight average molecular weight of more than 50000, the viscosity of the acidic polymer solution becomes high, and therefore it may be difficult to uniformly treat the polysiloxane coated ion sustained release glass. The concentration of the acidic polymer in the acidic polymer solution is preferably in the range from 3 to 25 % by mass, more preferably in the range from 8 to 20 % by mass. When the concentration of the acidic polymer is less than 3 % by mass, the above described acidic polymer reaction phase is brittle and therefore the effect of improving the sustained release of ions cannot be obtained. When the concentration of the acidic polymer is more than 25 % by mass, the polysiloxane layer (porous) is difficult to diffuse in a uniform state, a homogeneous acidic polymer reaction phase is not obtained, and the reaction occurs immediately after contact with the polysiloxane coated ion sustained release glass, and therefore there is a problem that strongly reacted agglomerates generates. The addition amount of the acidic polymer solution to the polysiloxane coated ion sustained release glass is preferably in the range from 6 to 40 % by mass, more preferably 10 to 30 % by mass. Converting in this addition amount, an optimal amount of the acid polymer with respect to the polysiloxane coated ion sustained release glass is in the range of 1 to 7 % by mass, and an optimal amount of water is in the range of 10 to 25 % by mass.

As the acid polymer that can be used to form the acid polymer reaction phase on the surface of the polysiloxane coated ion sustained release glass by the method described above, any copolymers or homopolymers as long as the copolymer or homopolymer is a copolymer or homopolymer of a polymerizable monomer having an acid group such as a phosphoric acid residue, a pyrophosphoric acid residue, a thiophosphoric acid residue, a carboxylic acid residue, or a sulfonic acid residue. Specific examples of these polymerizable monomers include acrylic acid, methacrylic acid, 2-chloroacrylic acid, 3-chloroacrylic acid, aconitic acid, mesaconic acid, maleic acid, itaconic acid, fumaric acid, glutaconic acid, citraconic acid, 4-(meta) acryloyloxy ethoxycarbonyl phthalic acid, 4-(meta) acryloyloxy ethoxycarbonyl phthalic anhydride, 5-(meta) acryloylamino pentylcarboxylic acid, 11-(meth) acryloyloxy-1,1-undecane dicarboxylic acid, 2-(meta) acryloyloxyethyl dihydrogen phosphate, 10-(meta) acryloyloxydecyl dihydrogen phosphate, 20-(meta) acryloyloxyeicosil dihydrogen phosphate, 1,3-di (meth) acryloyloxypropyl-2-dihydrogen phosphate, 2-(meta) acryloyloxyethyl phenyl phosphate, 2-(meta) acryloyloxyethyl-2'-bromoethyl phosphate, (meta) acryloyloxyethyl phenylphosphonate, [2-(meth) acryloyloxyethyl] pyrophosphate, 2-(meta) acryloyloxyethyl dihydrogen dithiophosphosphate and 10-(meta) acryloyloxydecyl dihydrogen thiophosphate. Of these polymers which are (co)polymer of these polymerizable monomers, a homopolymer or a copolymer of $\alpha$-$\beta$ unsaturated carboxylic acid that is relatively slow in acid-base reaction with an acid reactive element contained in the polysiloxane coated ion sustained release glass is preferable, and specific examples include an acrylic acid polymer, an acrylic acid-maleic acid copolymer, and an acrylic acid-itaconic acid copolymer.

The above described (E) filler can be treated with a surface treatment material represented by a silane coupling material in order to improve the affinity to the polymerizable monomer, the dispersability in the polymerizable monomer, and the mechanical strength and water resistance of the cured product. The surface treatment material and the surface treatment method are not particularly limited, and known methods such as a method of spraying the surface treatment material while stirring the powdery filler, a method of dispersing and mixing the filler and the surface treatment material in a solvent, and a method of supplying a vapor or gaseous silane coupling material to the filler surface, can be adopted without limitation. As a silane coupling material used for surface treatment of the filler, methyltrimethoxysilane, methyltriethoxysilane, methyltrichlorosilane, dimethyldichlorosilane, trimethylchlorosilane, vinyltrichlorosilane, vinyltriethoxysilane, vinyltris (2-methoxyethoxy) silane, 3-methacryloyloxypropyl trimethoxysilane, 3-chloropropyl trimethoxysilane, 3-glycidoxypropyl trimethoxysilane, 3-(meth) acryloxypropyl trimethoxysilane, 8-(meth) acryloxyoctyl trimethoxysilane, 11-(meth) acryloxiundecyl trimethoxysilane, hexamethyldisilazane and the like are preferable. In addition to the silane coupling material, surface treatment of the filler can be performed by a method using a titanate coupling material or an aluminate coupling material. The treatment amount of the surface treatment material in the filler is preferably 0.01 to 30 parts by mass, more preferably 0.5 to 20 parts by mass with respect to 100 parts by mass of the filler before treatment.

The shape of the (E) filler is not particularly limited, and any shape of the filler such as a spherical shape, a needle shape, a plate shape, a crushed shape or a scale shape can be used. The average particle diameter of the filler is preferably 0.01 $\mu$m to 50 $\mu$m, more preferably 0.01 $\mu$m to 30 $\mu$m, still more preferably 0.05 $\mu$m to 20 $\mu$m, and more preferably 0.1 $\mu$m to 10 $\mu$m.

The dental composite resin of the present invention may contain (e) hydrophobic inorganic fine particle as the (E) filler. It is preferable that the (e) hydrophobic inorganic fine particle in the present invention has an average primary particle diameter of 1 to 100 nm. The primary particle diameter indicates the diameter of one particle (primary particle) constituting the powder. In the present invention, the average particle diameter may be, for example, an average particle diameter calculated based on a volume-based grain size distribution measured by a laser diffraction type particle size distribution measuring device or the like, and may be measured by, for example, a laser diffraction type grain size measuring apparatus

(Microtrac MT3300EXII: NIKKISO Co., Ltd.). In addition, the primary particle diameter can be measured by dynamic light scattering particle size measurement and can be measured by using electron micrographs for the case that primary particles are strongly aggregated to form secondary particles. In the case of using particles having a relatively small particle diameter, because the specific surface area is large, it is expected to exhibit thixotropy with a small amount of compounding. On the other hand, in the case of using particles with an average particle diameter of 0.1 to 10 μm, a large amount may need to be added. Examples of a method for hydrophobizing a fine particle include surface treatment with a modified silicone oil such as dimethylsilicone oil and/or surface treatment with a silane coupling material which has an alkylsilyl group and may have a trimethylsilyl group, a dimethylsilyl group, a methylsilyl group or a (meth) acryloyl group having an alkyl chain having 3 or more and 18 or less carbon atoms.

As a carrier for the (e) hydrophobic inorganic fine particle, an inorganic particle is preferably used. Examples of such inorganic particle include silica, alumina, zirconia, titanium oxide, ytterbium fluoride, barium sulfate, and composites thereof. It is particularly preferable to use hydrophobized silica fine particle or hydrophobized alumina fine particle having an average primary particle diameter of 1 to 40 nm.

Specific examples of hydrophobic silica or alumina fine particles include Aerosil R972, Aerosil R974, Aerosil R976, Aerosil R711, Aerosil R7200, Aerosil R976S, Aerosil R202, Aerosil R812, Aerosil R812S, Aerosil R805, Aerosil AluC805, Aerosil R8200, Aerosil R104, Aerosil R106, Aerosil RY200, Aerosil RX200, Aerosil RX300, Aerosil RY200S, Aerosil RA200H, and Aerosil RA200HS, which are manufactured by Nippon Aerosil Co., Ltd. and commercially available under the trade name of Aerosil.

It is preferable that the compounding amount of the (e) hydrophobic inorganic fine particle with respect to 100 parts by mass of the (A) polymerizable monomer, is 0.5 to 30 parts by mass, preferably 1 to 20 parts by mass. When the compounding amount is less than 0.5 parts by mass with respect to 100 parts by mass of the (A) polymerizable monomer, there is a case where expression of rheological property in the dental composite resin is not expected, and when the compounding amount exceeds 30 parts by mass, there is a case that the photocurable dental composition is significantly thickened, resulting in poor operational feeling.

In the present invention it is preferable that the dental composite resin contains the (E) filler, and the total amount of the (E) filler and the (e) hydrophobic inorganic fine particle is 100 to 400 parts by weight with respect to 100 parts by weight of the (A) polymerizable monomer. The (E) filler has a larger average particle diameter size than the (e) hydrophobic inorganic fine particle. Furthermore, in the case of compounding a large amount of the (e) hydrophobic inorganic fine particle, there is a case where the viscosity of the dental composite resin increased, and the operability is reduced. Therefore, for the purpose of improving the strength of the dental composite resin, an inorganic filler having an average particle diameter of 0.1 to 10 μm is preferably used as the (E) filler because a viscosity-increasing effect per compounding amount is low. In addition, the (E) filler can be compounded for purposes other than increasing strength, such as imparting X-ray contrast, imparting ion release property, imparting color tone conformity, adjusting color tone toning, improving adhesive property and improving operability. When the total amount of the (E) filler and the (e) hydrophobic inorganic fine particle is 100 parts by mass or more with respect to 100 parts by mass of the (A) polymerizable monomer, an improvement in the strength of the cured dental composite resin can be expected.

The dental composite resin of the present invention may contain a chemical polymerization initiator. Specific examples of an organic peroxide as chemical polymerization initiator include diacyl peroxides, peroxy esters, dialkyl peroxides, peroxy ketals, ketone peroxides, peroxy dicarbonates, and hydro peroxides. Specific examples of diacyl peroxides include acetyl peroxide, isobutyryl peroxide, benzoyl peroxide, decanoyyl peroxide, 3,5,5-trimethylhexanoyl peroxide, 2,4-dichlorobenzoyl peroxide, and lauroyl peroxide and the like. Specific examples of peroxyesters include α-cumylperoxy neodecanoate, t-butylperoxy neodecanoate, t-butylperoxy pivalate, 2,2,4-trimethylpentyl peroxy-2-ethyl hexanoate, t-amylperoxy-2-ethylhexanoate, t-butylperoxy-2-ethylhexanoate, di-t-butylperoxy isophthalate, di-t-butylperoxy hexahydro terephthalate, t-butylperoxy-3,3,5-trimethyl hexanoate, t-butylperoxy acetate, t-butylperoxy benzoate and t-butylperoxy maleric acid. Specific examples of dialkyl peroxides include di-t-butyl peroxide, dicumyl peroxide, t-butylcumyl peroxide, 2,5-dimethyl-2,5-di (t-butylperoxy) hexane, 1,3-bis (t-butylperoxy isopropyl) benzene, 2,5-dimethyl-2,5-di (t-butylperoxy)-3-hexyne and the like. Specific examples of peroxyketals include 1,1-di (t-butylperoxy) cyclohexane, 2,2-di (t-butylperoxy) butane, and n-butyl-4,4-(t-butylperoxy) parerate, 1,1-di (t-amylperoxy) cyclohexane and the like. Specific examples of ketone peroxides include methyl ethyl ketone peroxide, methyl isobutyl ketone peroxide, methyl cyclohexanone peroxide, cyclohexanone peroxide and the like. Specific examples of peroxydicarbonates include di-3-methoxyperoxy dicarbonate, di-2-ethylhexylperoxy dicarbonate, bis (4-t-butylcyclohexyl) peroxy dicarbonate, diisopropylperoxy dicarbonate, di-n-propylperoxy dicarbonate, di-2-ethoxyethylperoxy dicarbonate, diallylperoxy dicarbonate and the like. Specific examples of hydroperoxides include 2,5-dimethyl hexane-2,5-dihydroperoxide, diisopropylbenzene hydroperoxide, cumene hydroperoxide, t-butyl hydroperoxide and 1,1,3,3-tetramethyl butylhydroperoxide.

In the dental composite resin of the present invention, the exemplified organic peroxides may be used alone or in combination of two or more. Among these organic peroxides, benzoyl peroxide and cumene hydroperoxide, 1,1,3,3-tetramethylbutyl hydroperoxide and 1,1,3,3-tetramethylbutyl peroxy-2-ethylhexanoate are preferable from the viewpoint of curability.

The compounding amount of the organic peroxide as a chemical polymerization initiator is preferably set to 0.1 to 5 parts by mass, more preferably set to 0.3 to 3 parts by mass with respect to 100 parts by mass of the total amount of the (A) polymerizable monomer from the viewpoint of improving the curability. When the compounding amount of the organic peroxide is more than 5 parts by mass, it may be difficult to ensure sufficient operation time. On the other hand, when the compounding amount of the organic peroxide is less than 0.1 parts by mass, there is a case in which mechanical strength is insufficient.

In the dental composite resin of the present invention, in order to improve the curability, a chemical polymerization accelerator may further be compounded. Examples of chemical polymerization accelerators include a transition metal compound of the group 4 in the periodic table, a thiourea derivative an aliphatic amine, an aromatic amine, a sulfinic acid and a salt thereof, a borate compound, a sulfur-containing reductive inorganic compound, a nitrogen-containing reductive inorganic compound, a barbituric acid derivative, a triazine compound, a halogen compound and the like. The compounding amount of the chemical polymerization accelerator is preferably 0.01 to 5 parts by mass, more preferably 0.1 to 3 parts by mass, with respect to 100 parts by mass of the total amount of the (A) polymerizable monomer.

The transition metal compound of the period 4 in the periodic table as a chemical polymerization accelerator refers to a metal compound of groups 3 to 12 of the period 4 in the periodic table, and specifically, each metal compound of scandium (Sc), titanium (Ti), vanadium (V), manganese (Mn), iron (Fe), cobalt (Co), nickel (Ni), copper (Cu), and zinc (Zn) can be used without any limitation. Although each of the above transition metal element may have a multiple valences, they can be added to the dental composite resin of the present invention as long as the valence is stable. Examples include Sc (trivalent), Ti (tetravalent), V (trivalent, tetravalent or pentavalent), Cr (divalent, trivalent or hexavalent), Mn (divalent to heptavalent), Fe (divalent or trivalent), Co (divalent or trivalent), Ni (divalent), Cu (monovalent or divalent), Zn (divalent). Specific examples of the transition metal compound include scandium iodide (trivalent) and the like as a scandium compound, titanium chloride (tetravalent), titanium tetraisopropoxide (tetravalent) and the like as titanium compounds, acetylacetone vanadium (trivalent), divanadium tetraoxide (tetravalent), vanadylacetyl acetonate (tetravalent), vanadium stearate oxide (tetravalent), vanadyl oxalate (tetravalent), vanazyl sulfate (tetravalent), oxobis (1-phenyl-1,3-butandionate) vanadium (tetravalent), bis (maltlate) oxovanadium (tetravalent), vanadium pentoxide (pentavalent), sodium metavanadate (pentavalent) and the like as a vanadium compound, manganese acetate (divalent), manganese naphthenate (divalent) and the like as manganese compounds, iron acetate (divalent), iron chloride (divalent), iron acetate (trivalent), iron chloride (trivalent) and the like as an iron compound, cobalt acetate (divalent), cobalt naphthenate (divalent) and the like as a cobalt compound, nickel chloride (divalent) and the like as a nickel compound, copper chloride (monovalent), copper bromide (monovalent), copper chloride (divalent), copper acetate (divalent) and the like as a copper compound, and zinc chloride (divalent), zinc acetate (divalent) and the like as a zinc compound.

Among these, a trivalent or tetravalent vanadium compound and a divalent copper compound are preferable. Among them, because of having higher polymerization accelerating ability, a trivalent or tetravalent vanadium compound is more preferable, and a tetravalent vanadium compound is most preferable. A plurality of kinds of these transition metal compounds in the period 4 in the periodic table may be used in combination, if necessary. The compounding amount of transition metal compound is preferably 0.0001 to 1 parts by mass with respect to 100 parts by mass of the total amount of the (A) polymerizable monomer. When the compounding amount is less than 0.0001 parts by mass, there is a case where the polymerization accelerating effect is insufficient, and when the compounding amount exceeds 1 part by mass, there is a case where it causes discoloration or gelation of the dental composite resin and the storage stability is lowered.

Any known thiourea derivatives can be used as the thiourea derivative as the chemical polymerization accelerator without any limitation. Specific examples of the thiourea derivatives include dimethylthiourea, diethylthiourea, tetramethylthiourea, (2-pyridyl) thiourea, N-methylthiourea, ethylenethiourea, N-allylthiourea, N-allyl-N'-(2-hydroxyethyl) thiourea, N-benzylthiourea, 1,3-dicyclohexyl thiourea, N,N'-diphenylthiourea, 1,3-di (p-tolyl) thiourea, 1-methyl-3-phenylthiourea, N-acetylthiourea, N-benzoylthiourea, diphenylthiourea, dicyclohexylthiourea and the like. Among these, (2-pyridyl) thiourea, N-acetylthiourea and N-benzoylthiourea are preferable. A plurality of kinds of these thiourea derivatives can be used in combination, if necessary. The compounding amount of the thiourea derivative is preferably 0.1 to 5 parts by mass with respect to 100 parts by mass of the total amount of the (A) polymerizable monomers. When the compounding amount is less than 0.1 parts by mass, there is a case where the ability as a polymerization accelerator is insufficient, and when the compounding amount exceeds 5 parts by mass, the storage stability may be lowered.

Examples of sulfinic acid and its salt include p-toluene sulfinic acid, sodium p-toluene sulfinate, potassium p-toluene sulfinate, lithium p-toluene sulfinate, calcium p-toluene sulfinate, benzenesulfinic acid, sodium benzene sulfinate, potassium benzene sulfinate, lithium benzenesulfinate, calcium benzenesulfinate, 2,4,6-trimethyl benzenesulfinic acid, sodium 2,4,6-trimethyl benzenesulfinate, potassium 2,4,6-trimethyl benzenesulfinate, lithium 2,4,6-trimethyl benzenesulfinate, calcium 2,4,6-trimethyl benzenesulfinate, 2,4,6-triethyl benzenesulfinic acid, sodium 2,4,6-triethyl benzenesulfinate, potassium 2,4,6-triethyl benzenesulfinate, lithium 2,4,6-triethyl benzenesulfinate, calcium 2,4,6-triethyl benzenesulfinate, 2,4,6-triisopropyl benzenesulfinic acid, sodium 2,4,6-triisopropyl benzenesulfinate, potassium 2,4,6-triisopropyl benzenesulfinate, lithium 2,4,6-triisopropyl benzenesulfinate, calcium 2,4,6-triisopropyl benzenesulfinate and the like. Among them, sodium benzenesulfinate, sodium p-toluenesulfinate, and sodium 2,4,6-triisopropyl benzenesulfinate are particularly preferable.

As the borate compound, specific examples of the borate compound having one aryl group in one molecule include trialkylphenylboron, trialkyl (p-chlorophenyl) boron, trialkyl (p-fluorophenyl) boron, trialkyl (3,5-bistrifluoro methyl) phenyl boron, trialkyl [3,5-bis (1,1,1,3,3,3-hexafluoro-2-methoxy-2-propyl) phenyl] boron, trialkyl (p-nitrophenyl) boron, trialkyl (m-nitrophenyl) boron, trialkyl (p-butylphenyl) boron, trialkyl (m-butylphenyl) boron, trialkyl (p-butyloxyphenyl) boron, trialkyl (m-butyloxyphenyl) boron, trialkyl (p-octyloxyphenyl) boron and trialkyl (m-octyloxyphenyl) boron (the alkyl group is at least one selected from the group consisting of n-butyl group, n-octyl group and n-dodecyl group etc.) and salts thereof (sodium salt, lithium salt, potassium salt, magnesium salt, tetrabutyl ammonium salt, tetramethyl ammonium salt, tetraethyl ammonium salt, methyl pyridinium salt, ethyl pyridinium salt, butyl pyridinium salt, methyl quinolinium salt, ethyl quinolinium salt, butyl quinolinium salt and the like). Specific examples of the borate compound having two aryl groups in one molecule include dialkyl diphenylboron, dialkyl di (p-chlorophenyl) boron, dialkyl di (p-fluorophenyl) boron, dialkyl di (3,5-bistrifluoro methyl) phenyl boron, dialkyl di [3,5-bis (1,1,1,3,3,3-hexafluoro-2-methoxy-2-propyl) phenyl] boron, dialkyl di (p-nitrophenyl) boron, dialkyl di (m-nitrophenyl) boron, dialkyl di (p-butylphenyl) boron, dialkyl di (m-butylphenyl) boron, dialkyl di (p-butyl oxyphenyl) boron, dialkyl di (m-butyl oxyphenyl) boron, dialkyl di (p-octyl oxyphenyl) boron and dialkyl di (m-octyl oxyphenyl) boron (the alkyl group is at least one selected from the group consisting of n-butyl group, n-octyl group and n-dodecyl group etc.) and salts thereof (sodium salt, lithium salt, potassium salt, magnesium salt, tetrabutyl ammonium salt, tetramethyl ammonium salt, tetraethyl ammonium salt, methyl pyridinium salt, ethyl pyridinium salt, butyl pyridinium salt, methyl quinolinium salt, ethyl quinolinium salt, butyl quinolinium salt and the like). Specific examples of the borate compound having three aryl groups in one molecule include monoalkyl triphenylboron, monoalkyl tri (p-chlorophenyl) boron, monoalkyl tri (p-fluorophenyl) boron, monoalkyl tri (3,5-bistrifluoro methyl) phenyl boron, monoalkyl tri [3,5-bis (1,1,1,3,3,3-hexafluoro-2-methoxy-2-propyl) phenyl] boron, monoalkyl tri (p-nitrophenyl) boron, monoalkyl tri (m-nitrophenyl) boron, monoalkyl tri (p-butylphenyl) boron, monoalkyl tri (m-butylphenyl) boron, monoalkyl tri (p-butyl oxyphenyl) boron, monoalkyl tri (m-butyl oxyphenyl) boron, monoalkyl tri (p-octyl oxyphenyl) boron and monoalkyl tri (m-octyl oxyphenyl) boron (the alkyl group is at least one selected from the group consisting of n-butyl group, n-octyl group and n-dodecyl group etc.) and salts thereof (sodium salt, lithium salt, potassium salt, magnesium salt, tetrabutyl ammonium salt, tetramethyl ammonium salt, tetraethyl ammonium salt, methyl pyridinium salt, ethyl pyridinium salt, butyl pyridinium salt, methyl quinolinium salt, ethyl quinolinium salt, butyl quinolinium salt and the like). Specific examples of the borate compound having four aryl groups in one molecule include tetraphenylboron, tetra kis (p-chlorophenyl) boron, tetra kis (p-fluorophenyl) boron, tetra kis (3,5-bistrifluoro methyl) phenyl boron, tetra kis [3,5-bis (1,1,1,3,3,3-hexafluoro-2-methoxy-2-propyl) phenyl] boron, tetra kis (p-nitrophenyl) boron, tetra kis (m-nitrophenyl) boron, tetra kis (p-butylphenyl) boron, tetra kis (m-butylphenyl) boron, tetra kis (p-butyl oxyphenyl) boron, tetra kis (m-butyl oxyphenyl) boron, tetra kis (p-octyl oxyphenyl) boron, tetra kis (m-octyl oxyphenyl) boron, (p-fluorophenyl) triphenylboron, (3,5-bis trifluoromethyl) phenyl triphenylboron, (p-nitrophenyl) triphenylboron, (m-butyl oxyphenyl) triphenylboron, (p-butyl oxyphenyl) triphenylboron, (m-octyl oxyphenyl) triphenylboron and (p-octyl oxyphenyl) triphenylboron, and salts thereof (sodium salt, lithium salt, potassium salt, magnesium salt, tetrabutyl ammonium salt, tetramethyl ammonium salt, tetraethyl ammonium salt, methyl pyridinium salt, ethyl pyridinium salt, butyl pyridinium salt, methyl quinolinium salt, ethyl quinolinium salt, butyl quinolinium salt and the like).

Among these aryl borate compounds, it is more preferable to use a borate compound having 3 or 4 aryl groups in one molecule from the viewpoint of storage stability. Further, these aryl borate compounds can be used alone or as a mixture of two or more.

Examples of the sulfur-containing reductive inorganic compound include sulfites, bisulfites, pyrosulfites, thiosulfates, thionates and dithionite. Specific examples include sodium sulfite, potassium sulfite, calcium sulfite, ammonium sulfite, sodium bisulfite, potassium bisulfite, 3-mercaptopropyl trimethoxysilane, 2-mercaptobenzoxazole, decanethiol, thiobenzoic acid and the like.

Examples of the nitrogen-containing reductive inorganic compound include nitrites, and specific examples include sodium nitrite, potassium nitrite, calcium nitrite, ammonium nitrite and the like.

Specific examples of barbituric acid derivative include salts (alkali metals or alkaline earth metals are preferred) of barbituric acid, 1,3-dimethyl barbituric acid, 1,3-diphenyl barbituric acid, 1,5-dimethyl barbituric acid, 5-butyl barbituric acid, 5-ethyl barbituric acid, 5-isopropyl barbituric acid, 5-cyclohexyl barbituric acid, 1,3,5-trimethyl barbituric acid, 1,3-dimethyl-5-ethyl barbituric acid, 1,3-dimethyl-n-butyl barbituric acid, 1,3-dimethyl-5-isobutyl barbituric acid, 1,3-dimethyl barbituric acid, 1,3-dimethyl-5-cyclopentyl barbituric acid, 1,3-dimethyl-5-cyclohexyl barbituric acid 1,3-dimethyl-5-phenyl barbituric acid, 1-cyclohexyl-1-ethyl barbituric acid, 1-benzyl-5-phenyl barbituric acid, 5-methyl barbituric acid, 5-propyl barbituric acid, 1,5-diethyl barbituric acid, 1-ethyl-5-methyl barbituric acid, 1-ethyl-5-isobutyl barbituric acid, 1,3-diethyl-5-butyl barbituric acid, 1-cyclohexyl-5-methyl barbituric acid, 1-cyclohexyl-5-ethyl barbituric acid, 1-cyclohexyl-5-octyl barbituric acid, 1-cyclohexyl-5-hexyl barbituric acid, 5-butyl-1-cyclohexyl barbituric acid, 1-benzyl-5-phenyl barbituric acid and thiobarbituric acids. Specifically, the salts of these barbituric acids include sodium 5-butyl barbiturate, sodium1,3,5-trimethyl barbiturate, sodium 1-cyclohexyl-5-ethyl barbiturate and the like.

Specific examples of the halogen compound include dilauryl dimethyl ammonium chloride, lauryl dimethyl benzyl

ammonium chloride, benzyl trimethyl ammonium chloride, tetramethyl ammonium chloride, benzyl dimethyl acetyl ammonium chloride, dilauryl dimethyl ammonium bromide and the like.

The dental composite resin of the present invention may not contain a chemical polymerization initiator and a chemical polymerization accelerator. The dental composite resin of the present invention may not contain a polymerization initiator system other than the photopolymerization system.

<Other component>

Further, the dental composite resin of the present invention may contain a component other than above described (A) to (E) components. For example, an excipient typified by fumed silica, benzophenone-based and benzotriazole-based ultraviolet absorbers, polymerization inhibitors such as hydroquinone, hydroquinone monomethyl ether and 2,5-ditershally butyl-4-methylphenol, chain transfer materials such as $\alpha$-alkylstyrene compound, mercaptan compound such as n-butyl mercaptan and n-octyl mercaptan, and terpenoid compound such as limonene, myrsen, $\alpha$-terpinene, $\beta$-terpinene, $\gamma$-terpinene, terpinoren, $\beta$-pinene and $\alpha$-pinene, metal supplementary material such as aminocarboxylic acid chelating agent and phosphonic acid chelating agent, discoloration inhibitors, antibacterial materials, coloring pigments, water and solvent that can be mixed with water in any ratio, and other additives conventionally known in the art may be added as necessary and as desired.

A preparing method of the dental composite resin of the present invention is not particularly limited. Examples of a general preparing method of a dental composite resin include a method which comprises preparing a matrix by mixing (A) polymerizable monomer, (B) photosensitizer, (C) photoacid generator and (D) photopolymerization accelerator in advance, kneading the matrix and (E) filler, and removing air bubbles under reduced pressure to prepare a uniform paste. In the present invention, it can be prepared by the above-described method without any problem.

The dental composite resin of the present invention may contain only (A) polymerizable monomer, (B) photosensitizer, (C) photoacid generator, (D) photopolymerization accelerator and (E) filler. Further, as the components other than (A) to (E), only one or more of the above-mentioned components may be contained.

The dental composite resin kit of the present invention includes at least two dental composite resins above-described. In the present invention, the two dental composite resins included in the dental composite resin kit, that is, the first dental composite resin and the second dental composite resin have a contrast ratio of 0.3 to 0.75 in the cured body, and the difference in the contrast ratio between the first dental composite resin and the second dental composite resin is 0.3 or less, in the case of measuring under a condition of a thickness of 1 mm.

A contrast ratio is a measure of representing a transparency. A contrast ratio is calculated from Y value of XYZ color system which is defined in JIS Z8701. Among the tristimulus values, Y relates to brightness. Specifically, in the present invention, the sample plate with a thickness of 1.0 mm is placed on a black or white background. The standard light C is irradiated and the Y value of the reflected light is measured. When the background is black, the Y is referred to Yb. When the background is white, the Y is referred to Yw. The contrast ratio (C) is obtained from Yb/Yw. When the C value is close to 1, the materials are opaqueness. When the C value is close to zero, the materials are transparency.

When the difference in the contrast ratio between the first dental composite resin and the second dental composite resin is more than 0.3, there is a room for achieving an aesthetic restoration similar to commercially available kits (enamel shade, body shades and the like) in the case of stacking the composite resin with the lower contrast ratio on the upper layer. However, in the case of switching the lower and upper layers, the shade of the composite resin with the higher contrast ratio located in the upper layer is dominant, therefore it is difficult to reproduce a wide range of shades. As a result of an intensive study for the problem, it has been found that when the difference in the contrast ratio is 0.3 or less, it is possible to reproduce a wide range of shades, even in the case of switching the lower and upper layers. In the present invention, the difference in the contrast ratio between the first dental composite resin and the second dental composite resin is preferably 0.25 or less, and more preferably 0.2 or less.

Furthermore, when each of the contrast ratio of the first dental composite resin and the second dental composite resin is not within a range of 0.3 to 0.75, it is impossible to reproduce a translucent to slightly opaque color similar to a natural tooth. In the present invention, each of the contrast ratio of the first dental composite resin and the second dental composite resin is preferably 0.35 to 0.75, and most preferably 0.35 to 0.7.

Furthermore, in the present invention, when it is defined that ($L_1$*, $a_1$*, $b_1$*) is a value of a color tone in a cured body having a thickness of 1 mm of the first dental composite resin in the L*a*b* space measured in the white background and ($L_2$*, $a_2$*, $b_2$*) is a value of a color tone in a cured body having a thickness of 1 mm of the second dental composite resin in the L*a*b* space measured in the white background, the color difference $\Delta$E, expressed by the following formula (1), is 2 or more.

$$\text{Formula (1): } \Delta E = \{(L_1^* - L_2^*)^2 + (a_1^* - a_2^*)^2 + (b_1^* - b_2^*)^2\}^{1/2}$$

When $\Delta E$ is less than 2, the color difference between a single-layer filling using only one dental composite resin and a multi-layer filling using both the first dental composite resin and the second dental composite resin is small, therefore it is difficult for an operator to recognize the color difference and it is impossible to reproduce a wide range of shades. In the present invention, $\Delta E$ must be 2 or more, preferably 3 or more, more preferably 4 or more, even more preferably 5 or more, and most preferably 10 or more.

In the dental composite resin kit of the present invention, the first dental composite resin and the second dental composite resin may satisfy a predetermined relationship between lightness difference and chroma difference in the cured body. Specifically, in a layered body in which a cured body having a thickness of 0.3 mm of one of the first dental composite resin and the second dental composite resin and a cured body having a thickness of 0.7 mm of the other of the first dental composite resin and the second dental composite resin are stacked, when it is defined that ($L_{S1}^*$, $a_{S1}^*$, $b_{S1}^*$) is a value of a color tone of the layered body in the L*a*b* space measured in the white background in a state that the cured body having a thickness of 0.3 mm is positioned at front side and ($L_{S2}^*$, $a_{S2}^*$, $b_{S2}^*$) is a value of a color tone of the layered body in the L*a*b* space measured in the white background in a state that the cured body having a thickness of 0.7 mm is positioned at front side, the lightness difference $\Delta L^*1$ expressed by the following formula (2) and the chroma difference $\Delta C^*1$ expressed by the following formula (3) may satisfy the relationship of the following formula (4), in the dental composite resin of the present invention.

$$\text{Formula (2): } \Delta L^*1 = |L_{S1}^* - L_{S2}^*|$$

$$\text{Formula (3): } \Delta C^*1 = |\{(a_{S1}^*)^2 + (b_{S1}^*)^2\}^{1/2} - \{(a_{S2}^*)^2 + (b_{S2}^*)^2\}^{1/2}|$$

$$\text{Formula (4): } \Delta L^*1 < \Delta C^*1$$

In general, it is considered most desirable in restorative treatment to match the lightness of the restoration to the lightness of the restored site. For this reason, the smaller the change in lightness when switching the cavity side and the front surface side of the composite resin during multi-layer filling, the easier it is for the operator to select the shade. On the other hand, to accommodate natural teeth with various shades, it is necessary to be able to reproduce a wide range of shades. Therefore, a certain degree of change in chroma is required when switching the cavity side and the front surface side of the composite resin during multi-layer filling. Specifically, as shown in formula equation (4), satisfying the relationship $\Delta L^*1 < \Delta C^*1$ is required, and satisfying the relationship $\Delta L^*1 < \Delta C^*1 - 0.5$ is preferable, and satisfying the relationship $\Delta L^*1 < \Delta C^*1 - 1.0$ is most preferable.

In this case, it is preferable that in a layered body in which a cured body having a thickness of 0.7 mm of one of the first dental composite resin and the second dental composite resin and a cured body having a thickness of 0.3 mm of the other of the first dental composite resin and the second dental composite resin are stacked, the first dental composite resin and the second dental composite resin satisfy a predetermined relationship between lightness difference and chroma difference in the cured body. That is, when it is defined that ($L_{S3}^*$, $a_{S3}^*$, $b_{S3}^*$) is a value of a color tone of the layered body in the L*a*b* space measured in the white background in a state that the cured body having a thickness of 0.3 mm is positioned at front side and ($L_{S4}^*$, $a_{S4}^*$, $b_{S4}^*$) is a value of a color tone of the layered body in the L*a*b* space measured in the white background in a state that the cured body having a thickness of 0.7 mm is positioned at front side, it is preferable that the lightness difference $\Delta L^*2$ expressed by the following formula (5) and the chroma difference $\Delta C^*2$ expressed by the following formula (6) satisfy the relationship of the following formula (7).

$$\text{Formula (5): } \Delta L^*2 = |L_{S3}^* - L_{S4}^*|$$

$$\text{Formula (6): } \Delta C^*2 = |\{(a_{S3}^*)^2 + (b_{S3}^*)^2\}^{1/2} - \{(a_{S4}^*)^2 + (b_{S4}^*)^2\}^{1/2}|$$

$$\text{Formula (7): } \Delta L^*2 < \Delta C^*2$$

In this case, with regard to formula (7), satisfying the relationship $\Delta L^*2 < \Delta C^*2 - 0.5$ is preferable, and satisfying the relationship $\Delta L^*2 < \Delta C^*2 - 1.0$ is most preferable.

In the present invention, it is preferable that at least one of the first dental composite resin and the second dental composite resin has a light diffusivity of 1 or more in the cured body having a thickness of 1 mm. The light diffusivity in the present invention is expressed as D, calculated using the following formula:

$$D = \{(I20/\cos 20°) + (I70/\cos 70°)\}/(2\times I0)$$

(In the formula, I represents the luminous intensity of light transmitted through the sample, and I0, I20 and I70 represent the luminous intensity (light intensity) at 0 degrees, 20 degrees and 70 degrees relative to the direction perpendicular to the sample plate (the direction of light incidence), respectively.)

Because the dental composite resin filled in a restoration area blends in with the surrounding shade more easily, the light diffusivity is preferably 1 or more, more preferably 19 or more, and most preferably 26 or more.

The dental composite resin kit of the present invention must include at least the first dental composite resin and the second dental composite resin described above, but may include other dental composite resin other than the first dental composite resin and the second dental composite resin described above.

Furthermore, in the dental composite resin kit of the present invention, it is preferable that all of the composite resins included in the kit satisfy the above descried relationship where the first dental composite resin and the second dental composite resin satisfy, with any of the other composite resins included in the kit.

Furthermore, when the dental composite resin kit of the present invention includes three or more dental composite resins, it is preferable that any one of dental composite resin satisfies the above descried relationship where the first dental composite resin and the second dental composite resin satisfy, with all other dental composite resins.

Hereinafter, example of the present invention are specifically described. However, the present invention is not intended to be limited to these Examples.

The materials used in Examples and Comparative examples and their abbreviations are listed below.

[(A) Polymerizable monomer]

Bis-GMA: 2,2-bis [4-(3-methacryloyloxy-2-hydroxypropoxy) phenyl] propane
UDMA: N,N-(2,2,4-trimethyl hexamethylene) bis [2-(aminocarboxy) ethanol] methacrylate
D2.6E: 2,2-bis (4-(meth) acryloyloxy polyethoxyphenyl) propane in which the average addition mole number of ethoxy groups is 2.6
NPG: neopentyl glycol dimethacrylate
TEGDMA: triethyleneglycol dimethacrylate
GDMA: glycerine dimethacrylate
MDP: 10-methacryloyloxydecyl dihydrogen phosphate

[(B) Photosensitizer]

CQ: camphorquinone
MAPO: 2,4,6-trimethyl benzoyl diphenyl phosphine oxide
BAPO: phenyl bis (2,4,6-trimethylbenzoyl) phosphine oxide

[(C) Photoacid generator]

<Salt of an anion having an organic group in which at least one H is substituted with F and one or more atoms of P, B, Al, S, and Ga, and an aryl iodonium cation>

C1: bis (4-tert-butylphenyl) iodonium nonafluorobutane sulfonate

[Chemical formula 2]

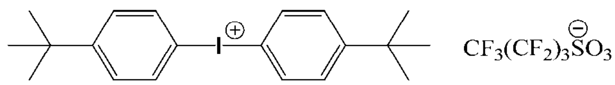

C2: bis (4-tert-butylphenyl) iodonium tris (pentafluoropropyl) trifluorophosphate

[Chemical formula 3]

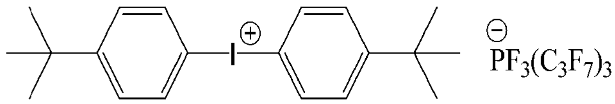

C3: p-cumenyl (p-tolyl) iodonium tris (pentafluoroethyl) trifluorophosphate

[Chemical formula 4]

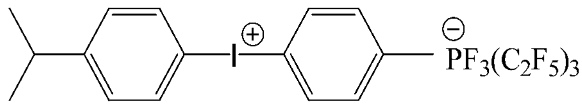

C4: p-cumenyl (p-tolyl) iodonium tetrakis (pentafluorophenyl) borate

[Chemical formula 5]

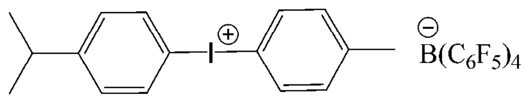

C5: bis [(4-tert-butyl) phenyl] iodonium tetra (nonafluoro-tert-butoxy) aluminate

[Chemical formula 6]

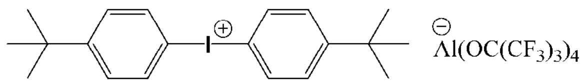

C6: bis [4-(tert-butyl) phenyl] iodonium tetra (pentafluorophenyl) gallate

[Chemical formula 7]

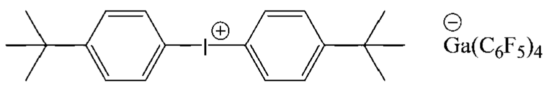

<Salt of an anion having an organic group and one or more atoms of P, B, Al, S, and Ga, and an aryl iodonium cation>
C11: bis (4-tert-butylphenyl) iodonium-p-toluenesulfonate

[Chemical formula 8]

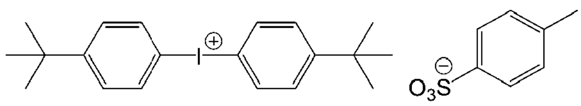

<Photoacid generator which is not a salt of an anion having an organic group and one or more atoms of P, B, Al, S, and Ga, and an aryl iodonium cation>

C21: diphenyl iodonium hexafluorophosphate

[Chemical formula 9]

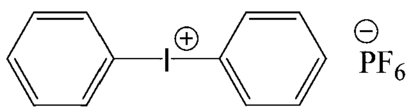

C22: 2,4,6-tris (trichloromethyl)-1,3,5-triazine

[Chemical formula 10]

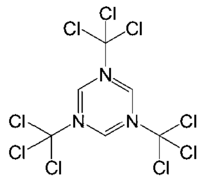

C23: diphenyliodonium-2-carboxylate monohydrate

[Chemical formula 11]

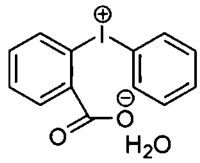

[(D) Photopolymerization Accelerator]

<Alphatic tertiary amine>

<<Aliphatic tertiary amine compound having no primary hydroxyl group>>

DMAEMA: N,N-dimethylamino ethylmethacrylate
DEAEMA: N,N-diethylamino ethylmethacrylate
TBA: tribenzylamine
PMPM: pentamethylpiperidyl methacrylate
DBAP: dibenzylaminopropanol

<<Aliphatic tertiary amine compound having two or more primary hydroxyl groups>>

MDEOA: methyl diethanolamine
TEA: triethanolamine

<Aromatic tertiary amine compound>
DMBE: N,N-dimethylaminobenzoic acid ethyl

[(E) Filler]

The preparing method of each filler used for preparing the dental compisite resin is shown below.

(Filler E1)

A silane coupling treatment solution prepared by stirring 50.0 g of water, 35.0 g of ethanol, and 3.0 g of 3-methacryloyloxypropyl trimethoxysilane as a silane coupling material at room temperature for 2 hours was added to 100.0 g of calcium fluoroaluminosilicate glass (average particle diameter: 1.1 μm) and stirred for 30 minutes. Thereafter, a heat treatment was performed at 100 °C for 15 hours to obtain a filler E1.

(Filler E2)

A silane coupling treatment solution prepared by stirring 50.0 g of water, 35.0 g of ethanol, and 5.0 g of 3-methacryloyloxypropyl trimethoxysilane as a silane coupling material at room temperature for 2 hours was added to 100.0 g of the zirconium silicate filler (average particle diameter: 0.8 μm, zirconia: 85 wt.%, silica:15 wt.%) and stirred for 30 minutes. Thereafter, a heat treatment was performed at 100 °C for 15 hours to obtain a filler E2.

(Filler E3)

After various raw materials of silica dioxide, aluminum oxide, boron oxide, sodium fluoride, and strontium carbonate were mixed, the mixture was melted at 1400 °C to obtain glass A (glass composition: $SiO_2$ 22.5% by mass, $Al_2O_3$ 20.0% by mass, $B_2O_3$ 12.3% by mass, SrO 35.7% by mass, $Na_2O$ 2.5% by mass, and F 7.0% by mass). The obtained glass A was pulverized using a vibration mill for 100 hours, and then pulverized using a wet bead mill for 3 hours. Then, 4.5 g of a low condensate of silane compound "MKC SILICATE MS56S" ($SiO_2$ content: 56.0% by mass, degree of polymerization: 2 to 100, manufactured by Mitsubishi Chemical Corporation) was added to 100 g of the obtained pulverized product, and stirred-mixed for 90 minutes. After mixing for a predetermined time, the resulting treated slurry was aged in a hot air dryer at 50 °C for 40 hours, then heated to 150 °C and held for 6 hours, then cooled to obtain a heat treated product. The obtained heat-treated product was placed in a Henschel mixer and pulverized at 1800 rpm for 5 minutes. After pulverization, a polysiloxane-treated material having good fluidity was obtained.

(Acidic polymer treatment)

Then, 100 g of the polysiloxane-treated product was put into a Henschel mixer, and 16.0 g of an aqueous polyacrylic acid solution (polymer concentration: 13% by mass, weight average molecular weight: 20,000; manufactured by Nacalai Co., Ltd.) was sprayed from above while stirring. After spraying, the powder taken out from the mixer was heated in a hot air dryer at 100 °C for 3 hours to obtain a polysiloxane-polyacrylic acid-treated product.

(Silane treatment)

A silane coupling treatment solution prepared by stirring 100.0 g of water, 80.0 g of ethanol, 0.003 g of phosphoric acid, and 12.0 g of 8-methacryloyloxypropyl trimethoxysilane as a silane coupling agent at room temperature for 2 hours was added to 100.0 g of the polysiloxane-polyacrylic acid-treated product and stirred for 30 minutes. Thereafter, a heat treatment was performed at 100 °C for 15 hours to obtain filler E3 (average particle diameter: 1.0 μm).

(Filler E4)

Filler E4 (average particle diameter 0.4 μm) was obtained by the same procedures as the Filler E3, except that the time for wet bead mill in the Filler E3 was extended.

(Filler E5)

After various raw materials of silica dioxide, aluminum oxide, boron oxide, sodium fluoride, and strontium carbonate were mixed, the mixture was melted at 1400 °C to obtain glass (glass composition: 22.5 % by massof $SiO_2$, 20.0 % by mass of $Al_2O_3$, 12.3 % by mass of $B_2O_3$, 35.7 % by mass of SrO, 2.5 % by mass of $Na_2O$, and 7.0 % by mass of F). The obtained glass was pulverized using a vibration mill for 100 hours, and then pulverized using a wet bead mill for 3 hours.

(Polysiloxane treatment)

Then, 4.5 g of a low condensate of silane compound "MKC SILICATE MS56S" (SiO2 content: 56.0% by mass, degree of polymerization: 2 to 100, manufactured by Mitsubishi Chemical Corporation) was added to 100 g of the obtained pulverized product, and stirred-mixed for 90 minutes. After mixing for a predetermined time, the resulting treated slurry was aged in a hot air dryer at 50 °C for 40 hours, then heated to 150 °C and held for 6 hours, then cooled to obtain a heat treated product. The obtained heat-treated product was placed in a Henschel mixer and pulverized at 1800 rpm for 5 minutes. After pulverization, a polysiloxane-treated material having a surface coated with polysiloxane and having good fluidity was obtained.

(Silane treatment)

A silane coupling treatment solution prepared by stirring 100.0 g of water, 80.0 g of ethanol, 0.003 g of phosphoric acid, and 12.0 g of 3-methacryloyloxypropyl trimethoxysilane as a silane coupling agent at room temperature for 2 hours was added to 100.0 g of the polysiloxane treated product and stirred for 30 minutes. Thereafter, a heat treatment was performed at 90 °C for 15 hours to obtain filler E5(average particle diameter of primary particle: 1.0 μm).

(Filler E6)

Filler E6 (average particle diameter 0.4 μm) was obtained by the same procedures as the Filler E3, except that the time for wet bead mill in the Filler E3 was extended.

(Filler E7)

Filler E7 (average particle diameter 3.0 μm) was obtained by the same procedures as the Filler E3, except that the time for wet bead mill in the Filler E3 was shortened.

(Filler E8)

Filler E8 (average particle diameter 10 μm) was obtained by the same procedures as the Filler E7, except that the time for wet bead mill in the Filler E7 was shortened.

<(e) Hydrophobic inorganic fine particle>

Filler e1: Aerosil R972 (Nippon Aerosil Co., Ltd.)
Filler e2: Aerosil R8200 (Nippon Aerosil Co., Ltd.)
Filler e3: Aerosil R711 (Nippon Aerosil Co., Ltd.)
Filler e4: Aerosil AluC805 (Nippon Aerosil Co., Ltd.)
Filler e5: Aerosil R805 (Nippon Aerosil Co., Ltd.)

[UV absorber]

BT: 2-(2-hydroxy-5-methylphenyl) benzotriazole

[Polymerization inhibitor]

MeHQ: p-methoxyphenol

[Fluorescent agent]

FA: 2.5-dihydroxyterephthalate diethyl

The test methods adopted in Examples and Comparative Examples are as follows.

(1) Color measurement of dental composite resin

A dental composite resin was filled into a mold (15φ × 1 mm: disk shape) made of stainless steel. Thereafter, a cover glass was placed on upper side of the stainless mold to apply pressure with glass plate. Subsequently, light irradiation was performed for 1 minute using a photopolymerization irradiator (Grip Light II, manufactured by SHOFU INC.) via the cover glass to prepare a cured material. The cured material was taken out of the mold, the cover glass was removed and the test specimen was measured for color tone. Color measurement was performed by placing the test specimen on the background of a standard white plate (D65/10°, X=81.07, Y=86.15, Z=93.38) and using a spectrocolorimeter (manufactured by BYK-Chemie GmbH) under predetermined condition (light source: C, viewing angle: 2°, measurement area: 11 mm).

Furthermore, when it is defined that (L1*, a1*, b1*) is a value of a color tone in a cured body having a thickness of 1 mm of the first dental composite resin in the L*a*b* space measured in the white background and (L2*, a2*, b2*) is a value of a color tone in a cured body having a thickness of 1 mm of the second dental composite resin in the L*a*b* space measured in the white background, the color difference ΔE is expressed by the following formula (1).

$$\text{Formula (1): } \Delta E = \{(L1^* - L2^*)^2 + (a1^* - a2^*)^2 + (b1^* - b2^*)^2\}^{1/2}$$

When ΔE is low, there is little difference in shade between single-layer filling and multi-layer filling of multiple colors, ΔE must be 2 or more, preferably 3 or more, more preferably 4 or more, even more preferably 5 or more.

(2) Color measurement assuming multi-layer filling

A dental composite resin was filled into a mold (15φ × 0.3 mm: disk shape) made of stainless steel. Thereafter, the excess dental composite resin is removed by was scraping off to the same thickness as the mold. Subsequently, light irradiation was performed for 1 minute using a photopolymerization irradiator (Grip Light II, manufactured by SHOFU INC.) in the state of opening the top to prepare a cured material. The cured product was taken out of the mold and fitted into a stainless steel mold (15φ × 1.0 mm: disk shape). On top of the cured product, a different composite resin from the cured product was filled and a cover glass was placed on upper side of the stainless mold to apply pressure with glass plate. Subsequently, light irradiation was performed for 1 minute using a photopolymerization irradiator (Grip Light II, manufactured by SHOFU INC.) via the cover glass to prepare a cured material. The cured material was taken out of the mold, the cover glass was removed and the test specimen was measured for color tone. Color measurement was performed by placing the test specimen on the background of a standard white plate (D65/10°, X=81.07, Y=86.15, Z=93.38) and using a spectrocolorimeter (manufactured by BYK-Chemie GmbH) under predetermined condition (light source: C, viewing angle: 2°, measurement area: 11 mm). The color measurement was performed twice, once on each side, with the front side and back side in reverse of the cured product prepared by stacking(the spectrophotometer side was the front side and the standard white plate side was the back side). The difference in lightness ΔL* and the difference in chroma ΔC* were calculated using the following formula.

$$\text{Formula (2): } \Delta L^{*}1 = |L_{S1}^{*} - L_{S2}^{*}|$$

$$\text{Formula (3): } \Delta C^{*}1 = |\{(a_{S1}^{*})^2 + (b_{S1}^{*})^2\}^{1/2} - \{(a_{S2}^{*})^2 + (b_{S2}^{*})^2\}^{1/2}|$$

The chroma C* is calculated by $C^* = \{(a^*)^2 + (b^*)^2\}^{1/2}$.

Among the surfaces facing each other in the stacking direction, the surface that was filled first in preparing the cured product is referred to as the reference surface, and the surface that was filled later is referred to as the stack surface. The color measurement result in the case of the reference surface is the front side is ($L_{S1}^*$, $a_{S1}^*$, $b_{S1}^*$). On the other hand, the color measurement result in the case of the reference surface is the back side is ($L_{S2}^*$, $a_{S2}^*$, $b_{S2}^*$). In general, it is considered most desirable in restorative treatment to match the lightness of the restoration to the lightness of the restored site. For this reason, the smaller the change in lightness when swapping the cavity side and the surface side of the composite resin during multi-layer filling, the easier it is for the operator to select the color tone. On the other hand, to accommodate natural teeth with various shades, it is necessary to be able to reproduce a wide range of shades. Therefore, a certain degree of change in chroma is required when swapping the cavity side and the surface side of the composite resin during multi-layer filling. Specifically, it is to satisfy following formula (4).

$$\text{Formula (4): } \Delta L^{*}1 < \Delta C^{*}1$$

Satisfying the relationship ΔL*1 < ΔC*1 - 0.5 is preferable, satisfying the relationship ΔL*1 < ΔC*1 - 0.6 is more preferable, satisfying the relationship ΔL*1 < ΔC*1 - 0.7 is furhter preferable, and satisfying the relationship ΔL*1 < ΔC*1 - 1.0 is most preferable.

(3) Measurement of contrast ratio

Prepared dental composite resin was fully filled into a mold (in a shape of a disc having a diameter of 15 mm and a thickness of 1 mm) made of stainless steel. Thereafter, a cover glass was placed on upper side of the stainless mold to apply pressure with glass plate. Subsequently, light irradiation was performed for 1 minute using a photopolymerization irradiator (Grip Light II, manufactured by SHOFU INC.) via the cover glass to prepare a cured material. The cured material was taken out of the mold, the cover glass was removed and the test specimen was measured for color tone. Color measurement was performed by placing the test specimen on the background of a standard white plate (D65/10°, X=81.07, Y=86.15, Z=93.38) and using a spectrocolorimeter (manufactured by BYK-Chemie GmbH) under predetermined condition (light source: C, viewing angle: 2°, measurement area: 11 mm) and the result Y value was used as Yw (white back ground). Furhter, color measurement was performed by placing the test specimen on the background of a standard black plate (D65/10°, X=0.0, Y=0.0, Z=0.0) and using a spectrocolorimeter (manufactured by BYK-Chemie GmbH) under predetermined condition (light source: C, viewing angle: 2°, measurement area: 11 mm) and the result Y value was used as Yb (black back ground).

The contrast ratio was expressed as C/N, calculated using the following formula.

$$C/N = Yb\ (\text{black background}) / Yw\ (\text{white background})$$

Because the C/N must be a value indicating translucent to slightly opaque, similar to a natural tooth, and C/N should be 0.3 to 0.75, is preferably 0.35 to 0.75, and is most preferably 0.35 to 0.7.

Furthermore, in the case of that the difference in contrast ratio between composite resins used for layering exceeds 0.3, when the composite resin with the lower contrast ratio is layered on top layer, is more than 0.3, there is a room for achieving an aesthetic restoration similar to commercially available kits (enamel shade, body shades and the like) in the case of stacking the composite resin with the lower contrast ratio on the upper layer. However, in the case of stacking layers in reverse order, the color tone of the composite resin with the higher contrast ratio located in the upper layer is dominant, therefore is difficult to reproduce a wide range of shades. As long as the difference in contrast ratio is 0.3 or less, a wide range of shades can be reproduced even in the case of stacking layers in reverse order. From this, the difference in contrast ratio is 0.3 or less, preferably 0.25 or less, and most preferably 0.2 or less.

(4) Spectroscopic measurement of transmitted light

The prepared dental composition was filled into a stainless steel mold (in a shape of a disc having a diameter of 15 mm and a thickness of 1 mm), and the cover glasses placed on both sides to press with a glass kneading plate. Thereafter, light was irradiated for 30 seconds to 5 locations per 1 sample by using the photopolymerization irradiator (Griplight manufactured by SHOFU INC.) to cure the paste. After curing, the cured product was taken out of the mold and attached to a measuring jig of a three-dimensional auto-goniophotometer (GP-200, manufactured by MURAKAMI COLOR RESEARCH LABORATORY) to measure the distribution of transmitted light intensity from +90° to -90° around the incident direction. Light diffusivity was expressed as D, calculated using the following formula:

$$D = \{(I20/\cos 20°) + (I70/\cos 70°)\}/(2\times I0)$$

(In the formula, I represents the luminous intensity of light transmitted through the sample, and I0, I20 and I70 represent the luminous intensity (light intensity) at 0 degrees, 20 degrees and 70 degrees relative to the direction perpendicular to the sample plate (the direction of light incidence), respectively.)

Because the dental composite resin filled in a restoration area blends in with the surrounding shade more easily, the light diffusivity is preferably 1 or more, more preferably 19 or more, and most preferably 26 or more.

<Preparing method of dental composite resin>

All components shown in Table 1 other than the filler (E) were put into a wide mouthed plastic container and mixed by using a mix rotor VMRC-5 under the condition of 100 rpm for 48 hours to prepare a matrix. Then, the matrix, the filler (E) and optional trace colorant were put into a kneader, stirred, and then defoamed under vacuum to prepare a paste like dental composite resin. In the Table 1, the content (parts by mass) of each component is indicated by the numerical value in parentheses after the abbreviation of each component.

[Table 1]

| Dental composite resin | (A) polymerizable monomer | (B) photosensitizer | (C) photoacid generator | (D) photopolymerization accelerator | (E) filler | | Polymerization inhibitor | Other |
|---|---|---|---|---|---|---|---|---|
| | | | | | (E) inorganic filler having an average primary particle diameter of 0.1 to 10 μm | (e) hydrophobic inorganic fine particle | | |
| Composition 1 | Bis-GMA(40),UDMA(20),NPG(40) | CQ(0.1) | C2(2) | DEAEMA(2) | E1(250) | e1(20) | MeHQ(0.005) | - |
| Composition 2 | Bis-GMA(60), TEGDMA(40) | CQ(0.2) | C3(2) | TBA(2) | E2(250), E3(10) | e2(20) | - | FA(0.01) |
| Composition 3 | Bis-GMA(60), TEGDMA(30), MDP(10) | CQ(0.3) | C1(1) | PMPM(2) | E8(200),E7(20), E3(5) | e3(20) | MeHQ(0.005) | FA(0.01) |
| Composition 4 | D2.6E(80), TEGDMA(20) | CQ(0.3) | C4(3) | DBAP(2) | E3(250) | e4(20) | MeHQ(0.005) | FA(0.01) |
| Composition 5 | Bis-GMA(40),UDMA(20),TEGDMA(40) | CQ(0.3) | C5(1.5) | MDEOA(1.5) | E4(280) | e5(15) | MeHQ(0.005) | FA(0.01) |
| Composition 6 | Bis-GMA(50),TEGDMA(20),GDMA(20),MDP(10) | CQ(0.2) | C6(1.5) | TEA(1.5) | E3(250) | e2(20) | MeHQ(0.005) | FA(0.01) |
| Composition 7 | D-2.6E(60), TEGDMA(40) | CQ(0.3) | C11(1.5) | DEAEMA(1) | E4(280) | e2(20) | MeHQ(0.005) | FA(0.01) |
| Composition 8 | D-2.6E(50), TEGDMA(40), MDP(10) | CQ(0.3) | C21(1.5) | PMPM(2) | E7(200),E5(20), E3(10) | e2(18) | MeHQ(0.005) | FA(0.01) |
| Composition 9 | D-2.6E(60), TEGDMA(20),GDMA(20) | CQ(0.3) | C22(2) | DBAP(1) | E5(200),E3(20), E7(30) | e2(15) | MeHQ(0.005) | FA(0.01) |
| Composition 10 | Bis-GMA(50),TEGDMA(20),GDMA(20),MDP(10) | CQ(0.3) | C23(1.5) | PMPM(1) | E2(200),E5(20), E3(60) | e2(20) | MeHQ(0.005) | FA(0.01) |
| Composition 11 | D-2.6E(70), TEGDMA(30) | CQ(0.01) | C2(6) | DMAEMA(6) | E5(230), E3(60) | e2(20) | MeHQ(0.02) | FA(0.01) |
| Composition 12 | Bis-GMA(60), TEGDMA(40) | MAPO(1.0) | | | E6(100), E4(100) | e2(20) | MeHQ(0.005) | FA(0.01) |
| Composition 13 | Bis-GMA(60), TEGDMA(40) | BAPO(1.0) | | | E3(230) | e2(20) | MeHQ(0.005) | FA(0.01) |
| Composition 14 | Bis-GMA(40),UDMA(20),TEGDMA(40) | CQ(0.4) | | DMBE(1) | E4(230) | e2(20) | MeHQ(0.005) | BT(0.1) |
| Composition 15 | Bis-GMA(40),UDMA(20),TEGDMA(40) | CQ(0.4) | | DMBE(1) | E4(230) | e2(20) | MeHQ(0.005) | BT(0.1) |

<Measurement of contrast Ratio, color Tone, and light diffusivity>

The contrast ratio, color tone, and light diffusion of the cured dental composite resins were measured using the methods described above. The measurement results are shown in Table 2. In Composition 13, the contrast ratio was 1.00

(opaque), and therefore light diffusion was not measured.

[Table 2]

| Dental composite resin | Contrast ratio | Color tone | | | Light diffusivity |
|---|---|---|---|---|---|
| | | L* | a* | b* | |
| Composition 1 | 0.39 | 79.6 | -2.5 | 4.1 | 0.0 |
| Composition 2 | 0.56 | 78.8 | 0.5 | 17.5 | 80.2 |
| Composition 3 | 0.56 | 77.2 | 1.3 | 17.9 | 77.8 |
| Composition 4 | 0.58 | 74.5 | 3.0 | 24.2 | 80.2 |
| Composition 5 | 0.59 | 70.0 | 5.1 | 29.2 | 78.2 |
| Composition 6 | 0.65 | 82.2 | -0.6 | 12.1 | 92.1 |
| Composition 7 | 0.65 | 79.7 | 1.0 | 19.2 | 91.9 |
| Composition 8 | 0.66 | 79.1 | 2.1 | 20.5 | 92.2 |
| Composition 9 | 0.73 | 64.1 | 4.5 | 28.8 | 80.0 |
| Composition 10 | 0.51 | 70.4 | 3.8 | 33.4 | 26.0 |
| Composition 11 | 0.63 | 65.9 | 6.2 | 31.1 | 75.9 |
| Composition 12 | 0.27 | 78.8 | -2.9 | 5.7 | 0.0 |
| Composition 13 | 0.76 | 87.0 | -3.1 | 5.9 | 94.1 |
| Composition 14 | 0.44 | 76.3 | -1.2 | 13.2 | 19.2 |
| Composition 15 | 0.31 | 78.6 | -2.8 | 6.0 | 1.1 |

<Evaluation of shade matching in single-layer filling>

The shade matching of each dental composite resin was evaluated when filled in a single layer. A Class 1 cavity (3 mm diameter, 2 mm depth) was formed in the center of the occlusal surface of artificial teeth (first molar: Veracia SA, manufactured by SHOFU INC.) of five shades (A1, A2, A3, A3.5, and A4). An adhesive treatment (CERA RESIN BOND, manufactured by SHOFU INC.) was then performed to the cavity. After adhesive treatment, the composite resin was filled into the cavity and the paste was cured using a light-curing irradiator. After curing, the cured product was polished, and five evaluators visually confirmed the shade match and rated it on a 5-point scale. The evaluation criteria are as follows. In Table 3, the average scores of the five evaluators are rounded to the nearest whole number.

5: The boundary between the artificial tooth and the filled composite resin was not clearly visible, and the shade blended in very well.

4: The boundary between the artificial tooth and the filled composite resin was visible, but the shade blended in.

3: The boundary between the artificial tooth and the filled composite resin was easily visible, but there is an acceptable shade match.

2: The boundary between the artificial tooth and the filled composite resin was easily visible, and the shade was slightly different.

1: The shade was clearly different between the artificial tooth and the filled composite resin.

When the shade of the cured body is close to the shade of the restored tooth, for example, when the evaluation result is 3 to 5, and the dental composite resin can be used in a single-layer filling, and it is also expected that it is used in a single-layer filling at the operator’s discretion.

[Table 3]

| Dental composite resin | Evaluation shade in filling artificial teeth | | | | |
|---|---|---|---|---|---|
| | A1 | A2 | A3 | A3.5 | A4 |
| Composition 1 | 3 | 2 | 2 | 2 | 2 |
| Composition 2 | 5 | 4 | 3 | 2 | 2 |
| Composition 3 | 4 | 5 | 4 | 3 | 2 |
| Composition 4 | 3 | 4 | 5 | 4 | 3 |
| Composition 5 | 2 | 3 | 4 | 5 | 4 |
| Composition 6 | 5 | 4 | 3 | 2 | 2 |
| Composition 7 | 5 | 4 | 3 | 2 | 2 |
| Composition 8 | 4 | 5 | 4 | 3 | 2 |
| Composition 9 | 2 | 2 | 2 | 3 | 4 |
| Composition 10 | 2 | 2 | 3 | 4 | 3 |
| Composition 11 | 2 | 2 | 3 | 4 | 5 |
| Composition 12 | 1 | 1 | 1 | 1 | 1 |
| Composition 13 | 1 | 1 | 1 | 1 | 1 |
| Composition 14 | 4 | 3 | 2 | 2 | 2 |
| Composition 15 | 3 | 2 | 2 | 2 | 2 |

Table 3 shows the relationship between each dental composite resin, artificial teeth of five shades (A1, A2, A3, A3.5, and A4), and evaluation results. It was confirmed that the compositions 1 to 11 and 14 which had contrast ratios in the range of 0.3 to 0.75, had good shade matching with any of the artificial teeth. In particular, in compositions 2 to 11 and 14 which were filled with dental composite resins with light-diffusing properties it was confirmed to have better shade matching.

On the other hand, compositions 12 and 13, which are dental composite resins with contrast ratio not within the range of 0.3 to 0.75, do not have a contrast ratio similar to that of a natural tooth, which is translucent to slightly opaque, and therefore have low shade matching with all artificial teeth A1 to A4 in single-layer fillings. For this reason, even if dental composite resins with such compositions are included in a dental composite resin kit, it will not be possible to prepare a dental composite resin kit that can reproduce a wide range of shades and perform aesthetic filling restorations, even if the number of dental composite resins is reduced.

<Result of color measurement assuming multi-layer filling >

According to the method described above in "Color measurement assuming multi-layer filling", two types of the cured bodies in which the dental composite resins according to the combination in the Examples and Comparative Examples in Tables 4 and 5 were stacked were prepared. The color tone of the resulting cured product was measured. In addition, $|\Delta L^*|$ and $|\Delta C^*|$ were calculated based on the measured color tone. Tables 4-6 show the measurement and calculation results. The calculation results shown in Tables 4-6 may not completely match the measurement results due to significant digits.

[Table 4]

| Example 1 | | Reference surface is front side | | | | Reference surface is the back side | | | | | |
|---|---|---|---|---|---|---|---|---|---|---|---|
| Reference surface | Stack surface | L* | a* | b* | C* | L* | a* | b* | C* | ⊿L* | ⊿C* |
| Composition 1 | Composition 2 | 78.1 | -0.8 | 15.6 | 15.6 | 78.2 | -0.6 | 14.9 | 14.9 | 0.0 | 0.7 |

(continued)

| Example 1 | | Reference surface is front side | | | | Reference surface is the back side | | | | | |
|---|---|---|---|---|---|---|---|---|---|---|---|
| Reference surface | Stack surface | L* | a* | b* | C* | L* | a* | b* | C* | ⊿L* | ⊿C* |
| Composition 2 | Composition 1 | 78.6 | -2.1 | 9.6 | 9.8 | 78.6 | -1.9 | 9.2 | 9.4 | 0.0 | 0.4 |
| | | | | | | | | | | | |
| Example 2 | | Reference surface is front side | | | | Reference surface is the back side | | | | | |
| Reference surface | Stack surface | L* | a* | b* | C* | L* | a* | b* | C* | ⊿L* | ⊿C* |
| Composition 1 | Composition 6 | 80.9 | -1.2 | 10.3 | 10.4 | 81.1 | -1.4 | 10.2 | 10.3 | 0.3 | 0.1 |
| Composition 6 | Composition 1 | 80.1 | -2.4 | 6.3 | 6.7 | 80.0 | -2.3 | 6.7 | 7.1 | 0.1 | 0.4 |
| | | | | | | | | | | | |
| Example 3 | | Reference surface is front side | | | | Reference surface is the back side | | | | | |
| Reference surface | Stack surface | L* | a* | b* | C* | L* | a* | b* | C* | ⊿L* | ⊿C* |
| Composition 1 | Composition 11 | 68.9 | 4.1 | 26.0 | 26.3 | 68.5 | 3.4 | 29.7 | 29.9 | 0.4 | 3.6 |
| Composition 11 | Composition 1 | 74.0 | -1.0 | 20.6 | 20.6 | 74.1 | 0.1 | 17.1 | 17.1 | 0.2 | 3.5 |
| | | | | | | | | | | | |
| Example 4 | | Reference surface is front side | | | | Reference surface is the back side | | | | | |
| Reference surface | Stack surface | L* | a* | b* | C* | L* | a* | b* | C* | ⊿L* | ⊿C* |
| Composition 2 | Composition 4 | 75.7 | 2.1 | 21.2 | 21.3 | 75.3 | 1.9 | 22.7 | 22.8 | 0.3 | 1.5 |
| Composition 4 | Composition 2 | 76.2 | 1.2 | 21.4 | 21.4 | 76.4 | 1.5 | 19.8 | 19.8 | 0.2 | 1.6 |
| | | | | | | | | | | | |
| Example 5 | | Reference surface is front side | | | | Reference surface is the back side | | | | | |
| Reference surface | Stack surface | L1* | a1* | b1* | C1* | L2* | a2* | b2* | C2* | ⊿L* | ⊿C* |
| Composition 2 | Composition 5 | 73.8 | 3.2 | 23.0 | 23.2 | 73.3 | 2.8 | 26.5 | 26.7 | 0.4 | 3.4 |
| Composition 5 | Composition 2 | 75.3 | 1.8 | 24.7 | 24.7 | 75.4 | 2.3 | 21.6 | 21.7 | 0.1 | 3.0 |
| | | | | | | | | | | | |

(continued)

| Example 6 | | Reference surface is front side | | | | Reference surface is the back side | | | | | |
|---|---|---|---|---|---|---|---|---|---|---|---|
| Reference surface | Stack surface | L1* | a1* | b1* | C1* | L2* | a2* | b2* | C2* | ⊿L* | ⊿C* |
| Composition 2 | Composition 7 | 79.0 | 0.8 | 19.6 | 19.6 | 79.2 | 0.7 | 18.6 | 18.6 | 0.2 | 1.0 |
| Composition 7 | Composition 2 | 78.9 | 0.5 | 18.3 | 18.3 | 78.6 | 0.6 | 19.1 | 19.1 | 0.3 | 0.9 |
| | | | | | | | | | | | |
| Example 7 | | Reference surface is front side | | | | Reference surface is the back side | | | | | |
| Reference surface | Stack surface | L1* | a1* | b1* | C1* | L2* | a2* | b2* | C2* | ⊿L* | ⊿C* |
| Composition 2 | Composition 8 | 79.0 | 1.5 | 20.2 | 20.3 | 79.2 | 1.3 | 19.7 | 19.7 | 0.2 | 0.6 |
| Composition 8 | Composition 2 | 78.8 | 0.9 | 18.5 | 18.6 | 78.4 | 1.0 | 19.3 | 19.3 | 0.3 | 0.8 |
| | | | | | | | | | | | |
| Example 8 | | Reference surface is front side | | | | Reference surface is the back side | | | | | |
| Reference surface | Stack surface | L1* | a1* | b1* | C1* | L2* | a2* | b2* | C2* | ⊿L* | ⊿C* |
| Composition 2 | Composition 11 | 68.6 | 5.1 | 26.2 | 26.7 | 67.9 | 4.9 | 31.1 | 31.5 | 0.7 | 4.7 |
| Composition 11 | Composition 2 | 73.3 | 2.2 | 27.4 | 27.5 | 73.5 | 2.9 | 22.9 | 23.0 | 0.2 | 4.5 |
| | | | | | | | | | | | |
| Example 9 | | Reference surface is front side | | | | Reference surface is the back side | | | | | |
| Reference surface | Stack surface | L1* | a1* | b1* | C1* | L2* | a2* | b2* | C2* | ⊿L* | ⊿C* |
| Composition 3 | Composition 4 | 74.5 | 2.9 | 21.7 | 21.9 | 74.2 | 2.8 | 23.0 | 23.1 | 0.2 | 1.3 |
| Composition 4 | Composition 3 | 76.7 | 1.8 | 20.3 | 20.4 | 76.5 | 1.9 | 18.9 | 19.0 | 0.2 | 1.3 |

[Table 5]

| Example 10 | | Reference surface is front side | | | | Reference surface is the back side | | | | | |
|---|---|---|---|---|---|---|---|---|---|---|---|
| Reference surface | Stack surface | L1* | a1* | b1* | C1* | L2* | a2* | b2* | C2* | ⊿L* | ⊿C* |
| Composition 3 | Composition 5 | 71.5 | 4.3 | 24.4 | 24.8 | 71.1 | 4.0 | 27.8 | 28.1 | 0.4 | 3.3 |
| Composition 5 | Composition 3 | 74.2 | 0.5 | 23.5 | 23.5 | 74.4 | 3.0 | 20.6 | 20.8 | 0.2 | 2.7 |
| | | | | | | | | | | | |

(continued)

| Example 11 | | Reference surface is front side | | | | Reference surface is the back side | | | | | |
|---|---|---|---|---|---|---|---|---|---|---|---|
| Reference surface | Stack surface | L1* | a1* | b1* | C1* | L2* | a2* | b2* | C2* | ⊿L* | ⊿C* |
| Composition 3 | Composition 11 | 68.3 | 5.3 | 26.4 | 26.9 | 67.8 | 5.1 | 30.9 | 31.3 | 0.5 | 4.4 |
| Composition 11 | Composition 3 | 71.9 | 3.1 | 27.8 | 27.9 | 72.4 | 3.7 | 23.1 | 23.4 | 0.5 | 4.6 |

| Example 12 | | Reference surface is front side | | | | Reference surface is the back side | | | | | |
|---|---|---|---|---|---|---|---|---|---|---|---|
| Reference surface | Stack surface | L1* | a1* | b1* | C1* | L2* | a2* | b2* | C2* | ⊿L* | ⊿C* |
| Composition 4 | Composition 6 | 79.0 | 0.6 | 19.2 | 19.2 | 79.8 | 0.9 | 15.2 | 15.2 | 0.8 | 4.0 |
| Composition 6 | Composition 4 | 77.1 | 2.0 | 18.4 | 18.5 | 76.5 | 1.8 | 22.5 | 22.6 | 0.7 | 4.0 |

| Example 13 | | Reference surface is front side | | | | Reference surface is the back side | | | | | |
|---|---|---|---|---|---|---|---|---|---|---|---|
| Reference surface | Stack surface | L1* | a1* | b1* | C1* | L2* | a2* | b2* | C2* | ⊿L* | ⊿C* |
| Composition 5 | Composition 6 | 77.3 | 1.2 | 21.8 | 21.8 | 78.4 | 1.5 | 15.4 | 15.5 | 1.1 | 6.3 |
| Composition 6 | Composition 5 | 74.7 | 3.3 | 19.7 | 19.9 | 73.5 | 3.0 | 27.1 | 27.2 | 1.2 | 7.3 |

| Example 14 | | Reference surface is front side | | | | Reference surface is the back side | | | | | |
|---|---|---|---|---|---|---|---|---|---|---|---|
| Reference surface | Stack surface | L1* | a1* | b1* | C1* | L2* | a2* | b2* | C2* | ⊿L* | ⊿C* |
| Composition 6 | Composition 11 | 71.0 | 4.5 | 21.1 | 21.6 | 69.2 | 4.5 | 31.3 | 31.6 | 1.8 | 10.0 |
| Composition 11 | Composition 6 | 74.4 | 1.9 | 27.1 | 27.2 | 76.2 | 2.4 | 17.1 | 17.3 | 1.7 | 9.9 |

| Example15 | | Reference surface is front side | | | | Reference surface is the back side | | | | | |
|---|---|---|---|---|---|---|---|---|---|---|---|
| Reference surface | Stack surface | L1* | a1* | b1* | C1* | L2* | a2* | b2* | C2* | ⊿L* | ⊿C* |
| Composition 7 | Composition 4 | 76.4 | 2.2 | 20.5 | 20.7 | 75.9 | 2.2 | 23.8 | 23.9 | 0.6 | 3.3 |
| Composition 4 | Composition 7 | 77.4 | 1.9 | 23.2 | 23.3 | 77.9 | 1.9 | 19.9 | 20.0 | 0.5 | 3.3 |

(continued)

| Example16 | | Reference surface is front side | | | | Reference surface is the back side | | | | | |
|---|---|---|---|---|---|---|---|---|---|---|---|
| Reference surface | Stack surface | L1* | a1* | b1* | C1* | L2* | a2* | b2* | C2* | ⊿L* | ⊿C* |
| Composition 7 | Composition 5 | 74.2 | 3.6 | 2.3 | 4.3 | 73.2 | 3.6 | 28.4 | 28.6 | 1.0 | 24.3 |
| Composition 5 | Composition 7 | 75.3 | 2.6 | 26.0 | 26.2 | 76.6 | 2.6 | 20.3 | 20.5 | 1.3 | 5.7 |
| | | | | | | | | | | | |
| Example17 | | Reference surface is front side | | | | Reference surface is the back side | | | | | |
| Reference surface | Stack surface | L1* | a1* | b1* | C1* | L2* | a2* | b2* | C2* | ⊿L* | ⊿C* |
| Composition 7 | Composition 11 | 71.1 | 4.6 | 23.3 | 23.7 | 69.3 | 4.9 | 31.6 | 31.9 | 1.8 | 8.2 |
| Composition 11 | Composition 7 | 74.9 | 2.5 | 27.6 | 27.7 | 75.9 | 2.7 | 20.9 | 21.0 | 1.0 | 6.6 |
| | | | | | | | | | | | |
| Example18 | | Reference surface is front side | | | | Reference surface is the back side | | | | | |
| Reference surface | Stack surface | L1* | a1* | b1* | C1* | L2* | a2* | b2* | C2* | ⊿L* | ⊿C* |
| Composition 5 | Composition 11 | 66.7 | 6.1 | 30.3 | 30.9 | 66.7 | 6.0 | 31.2 | 31.7 | 0.0 | 0.8 |
| Composition 11 | Composition 5 | 67.7 | 5.8 | 30.7 | 31.2 | 67.6 | 5.9 | 30.3 | 30.8 | 0.1 | 0.4 |

[Table 6]

| Example19 | | Reference surface is front side | | | | Reference surface is the back side | | | | | |
|---|---|---|---|---|---|---|---|---|---|---|---|
| Reference surface | Stack surface | L1* | a1* | b1* | C1* | L2* | a2* | b2* | C2* | ⊿L* | ⊿C* |
| Composition9 | Composition 14 | 70.3 | 0.7 | 24.2 | 24.2 | 70.3 | 1.4 | 22.1 | 22.2 | 0.1 | 2.0 |
| Composition 14 | Composition9 | 67.2 | 3.2 | 25.7 | 25.9 | 66.9 | 2.8 | 28.4 | 28.5 | 0.3 | 2.6 |
| | | | | | | | | | | | |
| Example20 | | Reference surface is front side | | | | Reference surface is the back side | | | | | |
| Reference surface | Stack surface | L1* | a1* | b1* | C1* | L2* | a2* | b2* | C2* | ⊿L* | ⊿C* |
| Composition 10 | Composition 2 | 75.2 | 1.4 | 26.3 | 26.4 | 74.8 | 2.0 | 22.7 | 22.8 | 0.4 | 3.6 |
| Composition 2 | Composition 10 | 72.0 | 3.2 | 27.7 | 27.9 | 71.9 | 2.7 | 30.8 | 30.9 | 0.0 | 3.1 |
| | | | | | | | | | | | |

(continued)

| Example21 | | Reference surface is front side | | | | Reference surface is the back side | | | | | |
|---|---|---|---|---|---|---|---|---|---|---|---|
| Reference surface | Stack surface | L1* | a1* | b1* | C1* | L2* | a2* | b2* | C2* | ⊿L* | ⊿C* |
| Composition 14 | Composition 4 | 74.6 | 1.4 | 20.6 | 20.7 | 74.5 | 1.2 | 20.8 | 20.9 | 0.10 | 0.17 |
| Composition 4 | Composition 14 | 74.3 | 0.2 | 17.8 | 17.8 | 74.3 | 0.3 | 17.7 | 17.7 | 0.0 | 0.1 |
| | | | | | | | | | | | |
| Example22 | | Reference surface is front side | | | | Reference surface is the back side | | | | | |
| Reference surface | Stack surface | L* | a* | b* | C* | L* | a* | b* | C* | ⊿L* | ⊿C* |
| Composition 15 | Composition 2 | 77.4 | -1.1 | 15.8 | 15.8 | 77.5 | -0.9 | 15.1 | 15.1 | 0.0 | 0.7 |
| Composition 2 | Composition 15 | 77.9 | -2.4 | 9.7 | 10.0 | 77.9 | -2.2 | 9.3 | 9.6 | 0.0 | 0.4 |
| | | | | | | | | | | | |
| Example23 | | Reference surface is front side | | | | Reference surface is the back side | | | | | |
| Reference surface | Stack surface | L* | a* | b* | C* | L* | a* | b* | C* | ⊿L* | ⊿C* |
| Composition 1 | Composition 5 | 72.5 | 2.7 | 21.8 | 21.9 | 72.4 | 1.9 | 24.7 | 24.8 | 0.1 | 2.9 |
| Composition 5 | Composition 1 | 75.4 | -1.1 | 16.4 | 16.4 | 75.9 | -0.2 | 14.3 | 14.3 | 0.6 | 2.1 |
| | | | | | | | | | | | |
| Comparative Example1 | | Reference surface is front side | | | | Reference surface is the back side | | | | | |
| Reference surface | Stack surface | L* | a* | b* | C* | L* | a* | b* | C* | ⊿L* | ⊿C* |
| Composition 2 | Composition 3 | 77.7 | 0.7 | 17.7 | 17.7 | 77.9 | 0.7 | 17.9 | 17.9 | 0.2 | 0.2 |
| Composition 3 | Composition 2 | 77.3 | 0.8 | 18.0 | 18.1 | 77.4 | 0.8 | 18.2 | 18.2 | 0.1 | 0.1 |
| | | | | | | | | | | | |
| Comparative Example2 | | Reference surface is front side | | | | Reference surface is the back side | | | | | |
| Reference surface | Stack surface | L1* | a1* | b1* | C1* | L2* | a2* | b2* | C2* | ⊿L* | ⊿C* |
| Composition 7 | Composition 8 | 79.7 | 1.7 | 19.8 | 19.9 | 79.5 | 1.8 | 20.1 | 20.2 | 0.2 | 0.3 |
| Composition 8 | Composition 7 | 79.7 | 1.4 | 19.8 | 19.8 | 79.7 | 1.5 | 19.5 | 19.6 | 0.0 | 0.3 |
| | | | | | | | | | | | |
| Comparative Example3 | | Reference surface is front side | | | | Reference surface is the back side | | | | | |
| Reference surface | Stack surface | L1* | a1* | b1* | C1* | L2* | a2* | b2* | C2* | ⊿L* | ⊿C* |
| Composition 1 | Composition9 | 68.0 | 3.0 | 23.3 | 23.5 | 67.3 | 2.1 | 28.0 | 28.0 | 0.7 | 4.5 |

(continued)

| Comparative Example3 | | Reference surface is front side | | | | Reference surface is the back side | | | | | |
|---|---|---|---|---|---|---|---|---|---|---|---|
| Reference surface | Stack surface | L1* | a1* | b1* | C1* | L2* | a2* | b2* | C2* | ⊿L* | ⊿C* |
| Composition9 | Composition 1 | 71.8 | -0.8 | 22.7 | 22.7 | 71.9 | 0.3 | 18.2 | 18.2 | 0.1 | 4.5 |
| | | | | | | | | | | | |
| Comparative Example4 | | Reference surface is front side | | | | Reference surface is the back side | | | | | |
| Reference surface | Stack surface | L1* | a1* | b1* | C1* | L2* | a2* | b2* | C2* | ⊿L* | ⊿C* |
| Composition 1 | Composition 12 | 78.4 | -2.5 | 5.3 | 5.9 | 78.6 | -2.5 | 5.3 | 5.8 | 0.1 | 0.0 |
| Composition 12 | Composition 1 | 78.7 | -2.3 | 4.5 | 5.0 | 78.6 | -2.2 | 4.4 | 4.9 | 0.1 | 0.1 |
| | | | | | | | | | | | |
| Comparative Example5 | | Reference surface is front side | | | | Reference surface is the back side | | | | | |
| Reference surface | Stack surface | L1* | a1* | b1* | C1* | L2* | a2* | b2* | C2* | ⊿L* | ⊿C* |
| Composition 13 | Composition 4 | 79.0 | 1.3 | 15.3 | 15.3 | 77.6 | 1.0 | 22.1 | 22.2 | 1.5 | 6.9 |
| Composition 4 | Composition 13 | 82.4 | -1.5 | 16.0 | 16.1 | 83.4 | -1.0 | 10.6 | 10.7 | 1.0 | 5.5 |

<Evaluation of shade matching in multi-layer filling>

A Class 1 cavity (3 mm diameter, 2 mm depth) was formed in the center of the occlusal surface of artificial teeth (first molar: Veracia SA, manufactured by SHOFU INC.) of five shades (A1, A2, A3, A4.5, and A4). An adhesive treatment (CERA RESIN BOND, manufactured by SHOFU INC.) was then performed to the cavity. After adhesive treatment, two composite resins were filled into the cavity and the paste was cured using a light-curing irradiator. After curing, the cured product was polished, and the shade matching was visually confirmed and rated it on a 5-point scale. In principle, it is easier to obtain good shade matching by filling the surface layer with a light-shade composite resin for a light-shade artificial tooth and filling the surface layer with a dark-shade composite resin for a dark-shade artificial tooth. However, in this evaluation, the thicknesses of the two (first and second) composite resins were changed between 3:7 (0.6 mm:1.4 mm) and 7:3 (1.4 mm:0.6 mm), and the upper/lower layers were switched, and the filling was performed a total of four times, and the evaluation of the best shade matching was adopted. As the evaluation value of the example, the lowest evaluation value among the evaluations of the five filled artificial teeth (A1, A2, A3, A4.5, and A4) as described above was recorded. The rating criteria are as follows.

5: The boundary between the artificial tooth and the filled composite resin was not clearly visible, and the shade blended in very well.

4: The boundary between the artificial tooth and the filled composite resin was visible, but the shade blended in.

3: The boundary between the artificial tooth and the filled composite resin was easily visible, but there is an acceptable shade match.

2: The boundary between the artificial tooth and the filled composite resin was easily visible, and the shade was slightly different.

1: The shade was clearly different between the artificial tooth and the filled composite resin.

For multiple restored teeth with different shades, it is expected that the shades can be adjusted by stacking and filling multiple dental composite resins.

[Table 7]

| | Dental composite resin lit | | ΔE (1mm) | Shade evaluation result | | | | | |
|---|---|---|---|---|---|---|---|---|---|
| | First dental composite resin | Second dental composite resin | | Evaluation of each filled artificial tooth | | | | | Rating Value (Minimum) |
| | | | | A1 | A2 | A3 | A3.5 | A4 | |
| Example 1 | Composition 1 | Composition 2 | 13.8 | 5 | 4 | 3 | 3 | 3 | 3 |
| Example 2 | Composition 1 | Composition 6 | 8.6 | 5 | 4 | 3 | 3 | 3 | 3 |
| Example 3 | Composition 1 | Composition 11 | 31.5 | 3 | 3 | 3 | 4 | 5 | 3 |
| Example 4 | Composition 2 | Composition 4 | 8.4 | 5 | 5 | 5 | 5 | 4 | 4 |
| Example 5 | Composition 2 | Composition 5 | 15.4 | 5 | 5 | 5 | 5 | 5 | 5 |
| Example 6 | Composition 2 | Composition 7 | 2.0 | 5 | 5 | 3 | 3 | 3 | 3 |
| Example 7 | Composition 2 | Composition 8 | 3.4 | 5 | 5 | 4 | 3 | 3 | 3 |
| Example 8 | Composition 2 | Composition 11 | 19.6 | 5 | 5 | 5 | 5 | 5 | 5 |
| Example 9 | Composition 3 | Composition 4 | 7.1 | 5 | 5 | 5 | 5 | 4 | 4 |
| Example 10 | Composition 3 | Composition 5 | 13.9 | 5 | 5 | 5 | 5 | 5 | 5 |
| Example 11 | Composition 3 | Composition 11 | 18.1 | 5 | 5 | 5 | 5 | 5 | 5 |
| Example 12 | Composition 6 | Composition 4 | 14.8 | 5 | 5 | 5 | 5 | 5 | 5 |
| Example 13 | Composition 6 | Composition 5 | 21.8 | 5 | 5 | 5 | 5 | 5 | 5 |
| Example 14 | Composition 6 | Composition 11 | 26.0 | 5 | 5 | 5 | 5 | 5 | 5 |
| Example 15 | Composition 7 | Composition 4 | 7.5 | 5 | 5 | 5 | 5 | 4 | 4 |
| Example 16 | Composition 7 | Composition 5 | 14.8 | 5 | 5 | 5 | 5 | 5 | 5 |
| Example 17 | Composition 7 | Composition 11 | 19.0 | 5 | 5 | 5 | 5 | 5 | 5 |
| Example 18 | Composition 5 | Composition 11 | 4.7 | 3 | 4 | 5 | 5 | 5 | 3 |
| Example 19 | Composition 9 | Composition 14 | 20.6 | 3 | 4 | 5 | 5 | 5 | 3 |
| Example 20 | Composition 10 | Composition 2 | 18.3 | 5 | 5 | 5 | 4 | 4 | 4 |

(continued)

| | Dental composite resin lit | | ΔE (1mm) | Shade evaluation result | | | | | |
|---|---|---|---|---|---|---|---|---|---|
| | First dental composite resin | Second dental composite resin | | Evaluation of each filled artificial tooth | | | | | Rating Value (Minimum) |
| | | | | A1 | A2 | A3 | A3.5 | A4 | |
| Example 21 | Composition 14 | Composition 4 | 11.9 | 4 | 5 | 5 | 4 | 4 | 4 |
| Example 22 | Composition 15 | Composition 2 | 12.0 | 5 | 5 | 4 | 3 | 3 | 3 |
| Example 23 | Composition 1 | Composition 5 | 27.9 | 3 | 3 | 4 | 5 | 3 | 3 |
| Comparative Example 1 | Composition 2 | Composition 3 | 1.9 | 5 | 5 | 4 | 3 | 2 | 2 |
| Comparative Example 2 | Composition 7 | Composition 8 | 1.8 | 5 | 5 | 4 | 3 | 2 | 2 |
| Comparative Example 3 | Composition 9 | Composition 1 | 30.0 | 2 | 2 | 3 | 4 | 2 | 2 |
| Comparative Example 4 | Composition 12 | Composition 1 | 1.8 | 2 | 1 | 1 | 1 | 1 | 1 |
| Comparative Example 5 | Composition 13 | Composition 4 | 23.1 | 2 | 2 | 3 | 2 | 2 | 2 |

Table 5 shows the results of the evaluation of shade matching in multi-layer filling. It was confirmed that all of Examples 1 to 23 had good shade matching. Among them, in Examples 4 to 22 which were filled with only a dental composite resin having light diffusivity, it was confirmed that the shade matching was superior to that of Examples 1 to 3 and 23 in which at least one dental composite resin was a dental composite resin that did not have light diffusivity.

On the other hand, in Comparative Examples 1 and 2 in which the ΔE between the two dental composite resins was less than 2, because the composite resins were similar in shade, there was little change in shade between single-layer filling and multi-layer filling, and it was confirmed that the shade matching for all five types of artificial teeth was not good.

In Comparative Example 3 in which the difference in C/N between the two dental composite resins exceeded 0.3, when the composition 9 which had a higher contrast ratio was stacked and filled on top, the shade of the composition 1 which has a lower contrast ratio less than 0.3 was difficult to be reflected, and the shade of the composition 9 became dominant, therefore it is impossible to reproduce a wide range of shades, and it was confirmed that the shade matching for all five types of artificial teeth was not good.

In Comparative Examples 4 and 5 which were filled with dental composite resins having a C/N not within the range of 0.3 to 0.75, the C/N was not a translucent to slightly opaque value similar to that of a natural tooth, and therefore the color matching when filled was low, and the shade is out of place and not blend in.

Furthermore, in Examples 1 and 3 to 23 in which ΔC* was larger than ΔL*, in the case of switching the top and bottom of the composite resins to be filled, the chroma changed but the change in lightness was suppressed, therefore operator can easily suppose the lightness, and therefore it is easy to reproduce a shade of tooth.

The dental composite resin of the present invention evaluated in the examples can be used without any problems with various known dental materials, such as a dental adhesive material, a dental core build-up material, a dental resin cement, a dental coating material, a dental sealant material, a dental manicure material, a dental splinting material, a dental hard resin, a dental CAD-CAM restoration material, a dental 3D printer material and the like.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

Industrial applicability

The present invention can provide to provide a dental composite resin kit which can reproduce a wide range of shades with a relatively small number of dental composite resins used in combination when performing aesthetic filling and restoration on teeth of various shades.

## Claims

1. A dental composite resin kit including at least a first dental composite resin and a second dental composite resin, wherein

    a contrast ratio of a cured body measured at a thickness of 1 mm of the first dental composite resin is 0.3 to 0.75,
    a contrast ratio of a cured body measured at a thickness of 1 mm of the second dental composite resin is 0.3 to 0.75,
    a difference in contrast ratio of a cured body between the first dental composite resin and the second dental composite resin is 0.3 or less, and
    when it is defined that ($L_1$*, $a_1$*, $b_1$*) is a value of a color tone in a cured body having a thickness of 1 mm of the first dental composite resin in the L*a*b* space measured in the white background and ($L_2$*, $a_2$*, $b_2$*) is a value of a color tone in a cured body having a thickness of 1 mm of the second dental composite resin in the L*a*b* space measured in the white background,
    the color difference ΔE expressed by the following formula (1) is 2 or more.

$$\text{Formula (1): } \Delta E = \{(L_1^* - L_2^*)^2 + (a_1^* - a_2^*)^2 + (b_1^* - b_2^*)^2\}^{1/2}$$

2. The dental composite resin kit according to claim 1, wherein

    in a layered body in which a cured body having a thickness of 0.3 mm of one of the first dental composite resin and the second dental composite resin and a cured body having a thickness of 0.7 mm of the other of the first dental composite resin and the second dental composite resin are stacked,
    when it is defined that ($L_{S1}$*, $a_{S1}$*, $b_{S1}$*) is a value of a color tone of the layered body in the L*a*b* space measured in the white background in a state that the cured body having a thickness of 0.3 mm is positioned at front side and ($L_{S2}$*, $a_{S2}$*, $b_{S2}$*) is a value of a color tone of the layered body in the L*a*b* space measured in the white background in a state that the cured body having a thickness of 0.7 mm is positioned at front side,
    the lightness difference ΔL*1 expressed by the following formula (2) and the chroma difference ΔC*1 expressed by the following formula (3) satisfy the relationship of the following formula (4) in the the first dental composite resin and the second dental composite resin.

$$\text{Formula (2): } \Delta L^*1 = |L_{S1}^* - L_{S2}^*|$$

$$\text{Formula (3): } \Delta C^*1 = |\{(a_{S1}^*)^2 + (b_{S1}^*)^2\}^{1/2} - \{(a_{S2}^*)^2 + (b_{S2}^*)^2\}^{1/2}|$$

$$\text{Formula (4): } \Delta L^*1 < \Delta C^*1$$

3. The dental composite resin kit according to claim 1, wherein at least one of the first dental composite resin and the second dental composite resin has a light diffusivity of 1 or more in the cured body having a thickness of 1 mm.

4. The dental composite resin kit according to claim 2, wherein

    in a layered body in which a cured body having a thickness of 0.3 mm of the other of the first dental composite resin and the second dental composite resin and a cured body having a thickness of 0.7 mm of the one of the first dental composite resin and the second dental composite are stacked,
    when it is defined that ($L_{S3}$*, $a_{S3}$*, $b_{S3}$*) is a value of a color tone of the layered body in the L*a*b* space measured in the white background in a state that the cured body having a thickness of 0.3 mm is positioned at front side and ($L_{S4}$*, $a_{S4}$*, $b_{S4}$*) is a value of a color tone of the layered body in the L*a*b* space measured in the white

background in a state that the cured body having a thickness of 0.7 mm is positioned at front side, the lightness difference $\Delta L^*2$ expressed by the following formula (5) and the chroma difference $\Delta C^*2$ expressed by the following formula (6) satisfy the relationship of the following formula (7) in the the first dental composite resin and the second dental composite resin.

$$\text{Formula (5): } \Delta L^*2 = |L_{S3}^* - L_{S4}^*|$$

$$\text{Formula (6): } \Delta C^*2 = |\{(a_{S3}^*)^2 + (b_{S3}^*)^2\}^{1/2} - \{(a_{S4}^*)^2 + (b_{S4}^*)^2\}^{1/2}|$$

$$\text{Formula (7): } \Delta L^*2 < \Delta C^*2$$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2024102444 A
- JP 2014148293 A
- JP 2016175851 A